# FASCICULE DE BREVET EUROPEEN

(11) **EP 3 856 063 B1**
(45) Date de publication et mention de la délivrance du brevet: **13.09.2023**
(21) Numéro de dépôt: 19795260.9
(22) Date de dépôt: 25.09.2019
(51) Int. Cl.: A61B 34/32, A61B 34/20, A61B 90/00, A61B 34/30, A61B 34/10

(54) **ROBOT MÉDICAL COMPORTANT DES MOYENS DE POSITIONNEMENT AUTOMATIQUE**
MEDIZINISCHER ROBOTER MIT AUTOMATISCHEN POSITIONIERUNGSMITTELN
MEDICAL ROBOT COMPRISING AUTOMATIC POSITIONING MEANS

(30) Priorité: 27.09.2018 FR 1858917; 01.03.2019 FR 1902154
(43) Date de publication de la demande: 04.08.2021
(73) Titulaire: Quantum Surgical, 34000 Montpellier (FR)
(72) Inventeur: BLONDEL, Lucien, 34070 MONTPELLIER (FR); BANEGAS, Frédéric, 34830 JACOU (FR); OLIVE, Sébastien, 34000 MONTPELLIER (FR); BADANO, Fernand, 69006 LYON (FR); NAHUM, Bertin, 34170 CASTELNAU-LE-LEZ (FR)
(74) Mandataire: Ipside
(86) Numéro de dépôt international: PCT/FR2019/052249
(87) Numéro de publication internationale: WO 2020/065209

(56) Documents cités:
- WO-A1-2017/076886
- WO-A1-2017/147596
- CN-A- 107 753 108
- FR-A1- 3 043 970
- US-A1- 2010 137 880
- US-A1- 2015 057 675
- US-A1- 2016 331 474
- US-A1- 2018 132 966

## Description

### DOMAINE TECHNIQUE

La présente invention appartient au domaine des robots médicaux destinés à assister un praticien lors d'une intervention médicale. Notamment, l'invention concerne un robot médical comportant des moyens de positionnement automatique dans une salle d'intervention, ainsi qu'un procédé de positionnement automatique d'un robot médical.

### ÉTAT DE LA TECHNIQUE

De nombreuses interventions médicales nécessitent de positionner ou de déplacer de manière très précise un outil médical par rapport à une anatomie d'intérêt d'un patient.

Par exemple, certaines interventions médicales nécessitent de placer un générateur d'ultrasons focalisés à haute intensité au contact de la peau du patient ou bien à l'intérieur du patient pour détruire une tumeur.

Selon un autre exemple, en neurochirurgie, certaines interventions médicales nécessitent d'introduire un cathéter, une aiguille ou une électrode dans une zone cible pour diagnostiquer ou traiter différentes pathologies.

Selon encore un autre exemple, en chirurgie orthopédique, il est fréquent pour le praticien de devoir positionner un foret de perçage, une fraise ou un autre instrument pour altérer une surface osseuse dans le but de réduire des fractures, corriger une difformité ou pallier une dégénérescence osseuse.

Pour certaines interventions décrites ci-dessus, il existe actuellement des robots médicaux permettant d'assister un praticien pour placer, maintenir ou guider un outil médical.

Un tel robot médical exécute généralement un plan d'intervention comportant une ou plusieurs actions à effectuer sur une anatomie d'intérêt d'un patient.

Un tel plan d'intervention est généralement déterminé pendant une phase de planification précédant l'intervention médicale, puis il est transmis au robot médical qui le mémorise par exemple dans la mémoire d'une unité de contrôle intégrée au robot médical et configurée pour diriger le robot médical.

La phase de planification peut être basée sur des images médicales de type scanner, tomodensitométrie, imagerie par résonance magnétique (IRM), tomographie par émission de positons (« positron émission tomography » ou PET en anglais), ultrasons, rayons X, etc. Le praticien peut alors choisir une position ou une trajectoire de l'outil médical par rapport à une anatomie d'intérêt du patient sur une ou plusieurs images médicales.

Dans certains cas, la phase de planification est basée sur une représentation 3D (en trois dimensions) de l'anatomie d'intérêt acquise par un système d'acquisition d'images non médicales ou par la construction d'un modèle géométrique de l'anatomie d'intérêt à partir de points ou surfaces collectées sur ladite anatomie d'intérêt.

Le robot médical doit être positionné à un endroit spécifique dans une salle d'intervention, à côté de la table sur laquelle est installé le patient pour lequel l'intervention médicale est prévue, de telle sorte que le robot médical puisse exécuter une ou plusieurs actions du plan d'intervention. Le robot médical porte un outil, par exemple un guide pour instrument médical ou un instrument médical monté à une extrémité d'un bras articulé, et il convient que la position du robot médical soit telle que le bras articulé puisse positionner ou déplacer l'outil dans toutes les positions ou trajectoires prévues dans le plan d'intervention. En effet, si tel n'était pas le cas, un repositionnement du patient ou du robot dans la salle d'intervention alors que l'ensemble des opérateurs et de l'équipement sont en conditions stériles pourrait s'avérer particulièrement laborieux et impliquer une perte de temps significative.

Le positionnement du robot médical est donc particulièrement important pour que les actions du plan d'intervention puissent être exécutées de manière précise.

Dans la majorité des cas, le positionnement du robot médical est une opération manuelle qui repose sur la formation et l'expérience de l'opérateur, ce qui peut entraîner des erreurs de positionnement ou des positionnements non optimaux du robot médical.

Dans certains cas, le robot médical est positionné de manière automatique à une position optimale pour exécuter le plan d'intervention. Dans la demande de brevet internationale WO2017/147596, notamment, le robot médical peut être positionné dans une position optimale à l'aide d'un système de suivi externe comportant des détecteurs optiques configurés pour détecter la position de marqueurs agencés sur des éléments de la salle d'intervention et sur le robot médical. La position du robot médical peut alors être définie relativement à ces marqueurs. Le système de suivi est par exemple disposé dans une lampe chirurgicale, un support mobile, les murs ou le plafond de la salle d'intervention. La détermination précise de la position optimale du robot médical est alors conditionnée par le bon positionnement du système de suivi et des différents marqueurs. Le système de suivi et les différents marqueurs étant installés par un opérateur, il y a des risques d'erreurs sur la position optimale du robot médical. Aussi, une cartographie de la salle d'intervention est utilisée par le système de suivi pour diriger le robot médical, et il convient de s'assurer que l'agencement de la salle d'intervention reste conforme à cette cartographie, ou bien que la cartographie soit mise à jour si l'agencement de la salle d'intervention change. En outre, le robot médical ne peut être utilisé que dans une salle d'intervention spécialement préparée et cartographiée pour que le système de suivi puisse diriger le robot médical.

Le besoin d'un système fiable et facile à installer et à maintenir est donc toujours présent pour positionner un robot médical à une position optimale dans une salle d'intervention, afin que le robot médical soit capable d'exécuter avec précision différentes actions d'un plan d'intervention sur une anatomie d'intérêt d'un patient. Les documents WO 2017/076886 A1 et US 2016/331474 A1 constituent également un état de l'art pertinent.

### EXPOSÉ DE L'INVENTION

La présente invention est définie par les revendications indépendantes 1 et 11 annexées. Des modes particuliers de mise en oeuvre de l'invention sont définis par les revendications dépendantes annexées. La présente invention a pour objectif de remédier à tout ou partie des inconvénients de l'art antérieur, notamment ceux exposés ci-avant, en proposant une solution permettant à un robot médical de se positionner de manière autonome à une position optimale dans une salle d'intervention quelconque pour pouvoir exécuter différentes actions d'un plan d'intervention sur un patient installé sur une table de ladite salle d'intervention. On entend par « salle d'intervention quelconque » que ladite salle n'est pas préparée spécifiquement pour que le robot médical soit fonctionnel dans cette salle, et que le robot médical ne possède a priori pas de cartographie de la salle ou d'informations relatives à l'agencement de la salle avant le début du procédé de positionnement du robot médical dans la salle d'intervention.

A cet effet, et selon un premier aspect, il est proposé par la présente invention, un procédé de positionnement automatique d'un robot médical pour réaliser une intervention médicale sur un patient. Le robot médical comporte une base mobile motorisée permettant de déplacer le robot médical, et une unité de contrôle mémorisant un plan d'intervention comportant au moins une action à effectuer sur une anatomie d'intérêt dudit patient. Le procédé de positionnement comporte les étapes suivantes, mises en oeuvre de manière autonome par le robot médical :
- une détection de la position de l'anatomie d'intérêt du patient par rapport à une position du robot médical,
- une identification, à partir de la position de l'anatomie d'intérêt du patient et du plan d'intervention, d'au moins deux positions favorables de la base mobile du robot médical auxquelles le robot médical est capable d'effectuer ladite au moins une action du plan d'intervention,
- un déplacement de la base mobile du robot médical à une position optimale sélectionnée parmi les positions favorables identifiées.

Dans la présente demande, le terme « médical » est à considérer au sens large et peut concerner aussi bien un contexte non chirurgical qu'un contexte chirurgical.

Un tel robot médical peut par exemple comporter un bras articulé avec plusieurs degrés de liberté à l'extrémité duquel peut être monté un outil, par exemple un guide pour instrument médical ou un instrument médical, tel qu'un générateur d'ultrasons, un guide pour cathéter, une aiguille, une électrode, une sonde, un bistouri, un foret de perçage, etc.

Selon un autre exemple, un tel robot médical peut par exemple être utilisé pour réaliser des images médicales (scanner, IRM, radiographie par rayons X, etc.). Dans un tel cas, le robot médical peut par exemple comporter un appareil d'imagerie médicale monté sur un support.

Le bras ou le support auquel est monté l'outil (guide pour instrument médical, instrument médical ou appareil d'imagerie médical) est solidaire de la base mobile motorisée du robot médical.

Le robot médical est configuré pour identifier de manière autonome au moins deux positions favorables de la base mobile du robot médical auxquelles le robot médical pourra exécuter chaque action d'un plan d'intervention.

Une position favorable de la base mobile du robot médical est déterminée en fonction de la position de l'anatomie d'intérêt du patient, du plan d'intervention, et éventuellement de la position d'une table d'une salle d'intervention sur laquelle le patient est installé.

Les positions du robot médical, de la table, de l'anatomie d'intérêt du patient, et éventuellement d'autres éléments de la salle d'intervention, sont définies dans un même repère spatial. Ces positions sont par exemple détectées par le robot médical par des moyens de repérage dans l'espace tels que des caméras, des télémètres, des capteurs inertiels, optiques ou odométriques, etc. Ces moyens de repérage dans l'espace sont portés par le robot médical, c'est-à-dire qu'ils sont solidaires de la base mobile du robot médical.

Il convient de noter que dans l'art antérieur la position de l'anatomie d'intérêt est parfois connue a priori par rapport à un référentiel de la salle d'intervention. Un simple changement de référentiel de cette position connue dans un référentiel du robot médical n'est pas une « détection » de la position d'intérêt au sens de l'invention. Dans l'invention, la position de l'anatomie d'intérêt du patient n'est pas connue a priori. Elle est complètement détectée par le robot médical pendant le procédé selon l'invention.

Par « anatomie d'intérêt » du patient, on entend une partie au moins du corps humain du patient pour laquelle un traitement est visé par le plan d'intervention.

Un plan d'intervention comporte une ou plusieurs actions à réaliser sur l'anatomie d'intérêt du patient. Une action du plan d'intervention correspond par exemple à pouvoir manipuler un outil dans un volume prédéterminé, à placer un outil d'intervention dans une position prédéterminée ou bien à déplacer cet outil selon une trajectoire prédéterminée par rapport à l'anatomie d'intérêt du patient. Selon un autre exemple, une action du plan d'intervention peut également consister à faire exécuter une opération particulière à l'outil, comme par exemple mettre en route un générateur d'ultrasons ou modifier l'intensité d'émission des ultrasons, ou bien déclencher la capture d'une image médicale. Ces exemples ne sont pas exhaustifs, et d'autres types d'actions sont bien évidemment envisageables. Une action du plan d'intervention est une action que le robot médical devra exécuter pendant l'intervention, ultérieurement au procédé de positionnement automatique du robot médical à une position optimale. L'exécution des actions du plan d'intervention ne fait donc pas partie du procédé de positionnement automatique du robot médical.

On entend qu'une étape du procédé de positionnement du robot médical est mise en oeuvre de manière autonome par le robot médical quand elle est exécutée par le robot médical, et lui seul, sans l'aide d'un autre dispositif ou opérateur humain. Cela signifie notamment que les informations provenant de capteurs utilisés pour identifier la ou les positions favorables sont fournies exclusivement par des capteurs portés par le robot médical.

Pour mettre en oeuvre le procédé de positionnement, le robot médical n'a pas besoin de posséder une cartographie de la salle d'intervention, ni de connaître des informations spécifiques sur l'agencement de la salle d'intervention, avant le début du procédé de positionnement.

L'ensemble de ces dispositions permettent de positionner automatiquement et précisément le robot médical par rapport à l'anatomie d'intérêt à traiter, de sorte que l'intervention médicale se déroule de façon la plus optimale possible. Le procédé de positionnement automatique d'un robot médical est ainsi facilité et fiabilisé.

Le positionnement automatique est facilité, notamment, car il n'y a pas besoin d'utiliser ou de maintenir un dispositif supplémentaire, comme par exemple un système de suivi qui aide à diriger et positionner le robot.

Le positionnement automatique est fiabilisé, notamment, car le procédé de positionnement automatique du robot médical selon l'invention limite les risques d'erreurs liés par exemple à l'intervention d'un opérateur humain ou à l'installation d'un dispositif supplémentaire nécessaire au pilotage du robot médical.

La position optimale du robot médical est déterminée à l'aide d'informations provenant de capteurs solidaires de la base mobile du robot médical, et non pas de capteurs externes au robot médical. La calibration des capteurs est ainsi facilitée et fiabilisée.

La position optimale de la base mobile du robot médical est celle qui permet un traitement le plus adapté possible de l'anatomie d'intérêt par le robot médical. La position optimale du robot médical ne peut pas toujours être définie à l'avance, car la position réelle du patient au moment de l'intervention ne correspond pas nécessairement à une position prévue ou modélisée pendant la phase de planification de l'intervention. Il est donc avantageux que le robot puisse se positionner automatiquement en fonction de la position de l'anatomie d'intérêt du patient au moment même de l'intervention, c'est-à-dire lorsque le patient est installé sur la table d'intervention et prêt à subir le traitement prévu dans le plan d'intervention.

Le procédé de positionnement automatique selon l'invention est également avantageux car le robot médical peut intervenir dans n'importe quelle salle d'intervention dans la mesure où il n'y a pas besoin de préparer la salle d'intervention spécifiquement pour que le robot médical puisse y intervenir. En effet, le robot médical n'a pas à connaître d'informations spécifiques sur la salle d'intervention, et notamment, il n'a pas à mémoriser une cartographie de la salle d'intervention avant le début du procédé de positionnement.

Comme il n'y a pas besoin de fournir de cartographie de la salle d'intervention au robot médical, il n'y a pas besoin de maintenir une telle cartographie. En particulier, il n'y a pas besoin de mettre à jour une telle cartographie si l'agencement de la salle d'intervention change. Cela réduit ainsi les tâches de maintenance relatives aux interventions médicales assistées par un robot médical. Cela permet également de limiter les risques d'erreurs liées par exemple au déplacement par inadvertance de certains éléments de la salle d'intervention qui auraient été cartographiés (par exemple des marqueurs) et dont la position ne correspondrait plus exactement à la position indiquée par la cartographie fournie au robot médical.

Aussi, avec le procédé de positionnement automatique selon l'invention, un même robot médical peut intervenir dans n'importe quelle salle d'intervention. Cela limite alors les coûts relatifs aux interventions médicales assistées par un robot car il n'y a pas besoin d'associer un robot médical à chaque salle d'intervention ou bien de préparer spécifiquement une salle d'intervention pour qu'un robot médical y soit fonctionnel.

Dans des modes particuliers de mise en oeuvre, l'invention peut comporter en outre l'une ou plusieurs des caractéristiques suivantes, prises isolément ou selon toutes les combinaisons techniquement possibles.

Dans des modes particuliers de mise en oeuvre, le patient est installé sur une table d'une salle d'intervention et le procédé comporte une détection de la position de la table. L'identification d'au moins une position favorable de la base mobile du robot médical est effectuée aussi à partir de la position de la table.

Dans des modes particuliers de mise en oeuvre, la détection de la position de la table et la détection de l'anatomie d'intérêt du patient sont effectuées à partir d'informations provenant de capteurs de repérage dans l'espace portés par le robot médical.

Une telle table est en effet un élément pouvant être identifié par le robot médical, par exemple en utilisant un algorithme de reconnaissance de forme exécuté par l'unité de contrôle du robot médical.

Dans des modes particuliers de mise en oeuvre, le robot est initialement dépourvu d'une cartographie de la salle d'intervention.

Dans des modes particuliers de mise en oeuvre, le procédé comporte en outre les étapes suivantes, mises en oeuvre de manière autonome par le robot médical :
- une génération d'une cartographie de la salle d'intervention sur laquelle sont représentées une position du robot médical, la position de la table, la position de l'anatomie d'intérêt du patient, et la position optimale,
- une détermination, à partir de ladite cartographie, d'un trajet du robot médical pour atteindre ladite position optimale.

Dans des modes particuliers de mise en oeuvre :
- le robot médical comporte un bras articulé et un outil monté à une extrémité dudit bras, une ou plusieurs articulations dudit bras étant contrôlées par l'unité de contrôle du robot médical pour positionner le bras selon une configuration possible,
- une position favorable correspond à une position de la base mobile du robot médical à laquelle il existe, pour chaque action du plan d'intervention, au moins une configuration possible du bras articulé permettant d'effectuer ladite action,
- le procédé comporte un calcul, pour chaque action du plan d'intervention et pour chaque position favorable identifiée, d'un paramètre d'accessibilité en fonction d'un nombre de configurations possibles du bras articulé pour effectuer ladite action lorsque la base mobile du robot médical est positionnée à ladite position favorable,
- la position optimale est sélectionnée en fonction des paramètres d'accessibilité des positions favorables identifiées.

Il est alors possible, par exemple, de sélectionner la position optimale comme étant celle pour laquelle le paramètre d'accessibilité pour une action particulière du plan d'action est le plus grand.

Dans des modes particuliers de mise en oeuvre, le paramètre d'accessibilité est calculé en outre en fonction de débattements disponibles des articulations du bras pour chacune desdites configurations possibles du bras. Dans des modes particuliers de mise en oeuvre :
- le procédé comporte en outre un calcul, pour chaque position favorable identifiée, d'un niveau global d'accessibilité du plan d'intervention, ledit niveau global d'accessibilité étant calculé en fonction des paramètres d'accessibilité de chaque action du plan d'intervention lorsque la base mobile du robot médical est positionnée à ladite position favorable,
- la position optimale est sélectionnée en fonction des niveaux globaux d'accessibilité calculés pour les positions favorables identifiées.

Il est alors possible de définir la position optimale de la base mobile du robot médical comme étant celle qui présente le niveau global d'accessibilité le plus grand parmi les différentes positions favorables identifiées.

Le niveau global d'accessibilité pour une position favorable identifiée peut par exemple être défini comme étant la somme des paramètres d'accessibilité de chaque action du plan d'intervention lorsque la base mobile du robot médical est positionnée à ladite position favorable. Le niveau global d'accessibilité peut également être défini avec des pondérations des paramètres d'accessibilité en fonction de l'importance d'une action relativement à d'autres actions du plan d'intervention

De telles dispositions permettent une plus grande flexibilité pendant l'intervention. En effet, le praticien pourra potentiellement choisir, une fois le robot positionné à la position optimale, pendant l'intervention, entre plusieurs configurations possibles pour effectuer telle ou telle action du plan d'intervention. La préférence d'une configuration particulière par rapport à une autre pourra dépendre du déroulement de l'intervention.

Dans des modes particuliers de mise en oeuvre, une action du plan d'intervention correspond à permettre de déplacer l'outil dans un volume prédéterminé par rapport à ladite anatomie d'intérêt du patient.

Dans des modes particuliers de mise en oeuvre, une action du plan d'intervention correspond à permettre le déplacement de l'outil selon une trajectoire prédéterminée par rapport à ladite anatomie d'intérêt du patient.

Dans des modes particuliers de mise en oeuvre, une action du plan d'intervention correspond à placer l'outil dans une position prédéterminée par rapport à ladite anatomie d'intérêt du patient. Selon l'invention, au moins deux positions favorables différentes sont identifiées et le procédé comporte les étapes suivantes :
- une vérification d'un critère de stabilité de la base mobile du robot médical à la position optimale sélectionnée,
- si ladite vérification est positive, une immobilisation de la base mobile du robot médical à ladite position optimale,
- si ladite vérification est négative, un déplacement de la base mobile du robot médical à une autre position sélectionnée parmi les positions favorables.

Dans des modes particuliers de mise en oeuvre, le robot médical comporte des pieds rétractables destinés à être abaissés pour immobiliser la base mobile du robot médical, chaque pied comportant un capteur permettant de mesurer un défaut local de planéité du sol en regard dudit pied, et la vérification d'un critère de stabilité comporte, pour chaque pied, une comparaison de la mesure du défaut local de planéité du sol pour ledit pied avec un seuil prédéterminé. Selon l'invention, le robot médical comporte des pieds rétractables destinés à être abaissés pour immobiliser la base mobile du robot médical, chaque pied comportant un capteur permettant de mesurer un poids supporté par ledit pied, et la vérification d'un critère de stabilité comporte, pour chaque pied, une comparaison de la mesure du poids supporté par ledit pied avec un seuil prédéterminé.

Dans des modes particuliers de mise en oeuvre, la position optimale est sélectionnée en fonction de préférences d'un opérateur mémorisées dans l'unité de contrôle.

De telles dispositions permettent d'adapter la position du robot médical aux besoins spécifiques d'une intervention ou aux préférences d'un opérateur, comme par exemple la position prévue du patient sur la table (décubitus ventral, dorsal ou latéral), la préférence d'un côté de la table pour installer le robot, la préférence de latéralité du praticien (selon que le praticien est droitier ou gaucher), etc.

Dans des modes particuliers de mise en oeuvre, la position optimale est sélectionnée par un algorithme d'apprentissage automatique en fonction d'informations extraites d'une base de données d'interventions médicales déjà réalisées.

Dans des modes particuliers de mise en oeuvre, ledit algorithme d'apprentissage automatique utilise un réseau de neurones multicouches.

Une position optimale déterminée sur la base d'un plan d'intervention construit à partir de modèles théoriques ne prenant pas en compte la réalité d'interventions déjà réalisées peut être significativement différente d'une position optimale déterminée en prenant en compte des éléments observés au cours d'interventions passées, notamment si la modélisation théorique du plan d'intervention est complexe. Il est ainsi avantageux d'utiliser des algorithmes d'apprentissage automatique prenant en compte des informations relatives à des interventions déjà réalisées.

Dans des modes particuliers de mise en oeuvre, le robot médical comporte une interface utilisateur, la ou les positions favorables identifiées sont proposées à un opérateur via ladite interface utilisateur, et la position optimale est sélectionnée par ledit opérateur.

Selon un deuxième aspect, la présente invention concerne un robot médical comportant une base mobile motorisée, des capteurs de repérage dans l'espace, et une unité de contrôle mémorisant un plan d'intervention comportant au moins une action à réaliser sur une anatomie d'intérêt d'un patient. L'unité de contrôle est configurée pour :
- détecter, à partir d'informations provenant des capteurs de repérage dans l'espace, une position de l'anatomie d'intérêt du patient par rapport au robot médical,
- identifier, à partir de la position de l'anatomie d'intérêt du patient et du plan d'intervention, au moins deux positions favorables de la base mobile du robot médical pour lesquelles le robot médical est capable d'effectuer ladite au moins une action du plan d'intervention,
- déplacer la base mobile du robot médical à une position optimale sélectionnée parmi les positions favorables identifiées.

Dans des modes particuliers de réalisation, l'invention peut comporter en outre l'une ou plusieurs des caractéristiques suivantes, prises isolément ou selon toutes les combinaisons techniquement possibles.

Dans des modes particuliers de réalisation, l'unité de contrôle est également configurée pour détecter, à partir d'informations provenant des capteurs de repérage dans l'espace, une position d'une table d'une salle d'intervention sur laquelle est installé le patient, et pour identifier la ou les positions favorables en fonction de la position de la table.

Dans des modes particuliers de réalisation, l'unité de contrôle est également configurée pour :
- générer une cartographie de la salle d'intervention sur laquelle sont représentées une position du robot médical, la position de la table, la position de l'anatomie d'intérêt du patient, et la position optimale,
- déterminer, à partir de ladite cartographie, un trajet du robot médical pour atteindre ladite position optimale.

Dans des modes particuliers de réalisation, le robot médical comporte en outre des capteurs d'aide à la détection de l'anatomie d'intérêt du patient coopérant avec des marqueurs positionnés sur ladite anatomie d'intérêt.

De tels marqueurs peuvent par exemple générer des signaux lumineux ou électromagnétiques permettant à des capteurs spécifiques d'estimer une distance entre le robot médical et l'anatomie d'intérêt.

Dans des modes particuliers de réalisation, l'unité de contrôle identifie ladite au moins une position favorable exclusivement à partir du plan d'intervention et d'informations provenant de capteurs portés par le robot médical.

Dans des modes particuliers de réalisation, le robot médical comporte un bras articulé et un outil monté à une extrémité dudit bras. Une ou plusieurs articulations du bras sont contrôlées par l'unité de contrôle du robot médical pour positionner le bras dans une configuration possible. Une position favorable correspond alors à une position de la base mobile du robot médical à laquelle il existe, pour chaque action du plan d'intervention, au moins une configuration possible du bras articulé pour effectuer ladite action. L'unité de contrôle est configurée pour :
- calculer, pour chaque action du plan d'intervention et pour chaque position favorable identifiée, un paramètre d'accessibilité en fonction d'un nombre de configurations possibles du bras articulé pour effectuer ladite action lorsque la base mobile du robot médical est positionnée à ladite position favorable,
- sélectionner la position optimale en fonction des paramètres d'accessibilité des positions favorables identifiées.

Selon l'invention, l'unité de contrôle est en outre configurée pour :
- vérifier un critère de stabilité de la base mobile du robot médical à la position optimale sélectionnée,
- si ladite vérification est positive, déclencher une immobilisation de la base mobile du robot médical à ladite position optimale,
- si ladite vérification est négative, déplacer la base mobile du robot médical à une autre position sélectionnée parmi les positions favorables.

Dans des modes particuliers de réalisation, le robot médical comporte des pieds rétractables destinés à être abaissés pour immobiliser la base mobile du robot médical, chaque pied comportant un capteur permettant de mesurer un défaut local de planéité du sol en regard dudit pied, et pour vérifier le critère de stabilité, l'unité de contrôle compare, pour chaque pied, la mesure du défaut local de planéité du sol pour ledit pied avec un seuil prédéterminé.

Selon l'invention, le robot médical comporte des pieds rétractables destinés à être abaissés pour immobiliser la base mobile du robot médical, chaque pied comportant un capteur permettant de mesurer un poids supporté par ledit pied, et pour vérifier le critère de stabilité, l'unité de contrôle compare, pour chaque pied, la mesure du poids supporté par ledit pied avec un seuil prédéterminé.

### PRÉSENTATION DES FIGURES

L'invention sera mieux comprise à la lecture de la description suivante, donnée à titre d'exemple nullement limitatif, et faite en se référant aux figures 1 à 5 qui représentent :
- Figure 1 : une représentation schématique d'un robot médical dans une salle d'intervention comportant une table sur laquelle est positionné un patient,
- Figure 2 : une représentation schématique détaillée d'un robot médical selon l'invention,
- Figure 3 : une représentation schématique d'une cartographie d'une salle d'intervention générée par le robot médical et d'un trajet à emprunter pour atteindre une position optimale pour traiter le patient,
- Figure 4 : les principales étapes d'un mode particulier de mise en oeuvre d'un procédé de positionnement automatique d'un robot médical selon l'invention,
- Figure 5 : les principales étapes d'un mode particulier de mise en oeuvre d'une sélection d'une position optimale d'un robot médical parmi plusieurs positions favorables.

Dans ces figures, des références identiques d'une figure à une autre désignent des éléments identiques ou analogues. Pour des raisons de clarté, les éléments représentés ne sont pas nécessairement à une même échelle, sauf mention contraire.

### DESCRIPTION DÉTAILLÉE DE MODES DE RÉALISATION

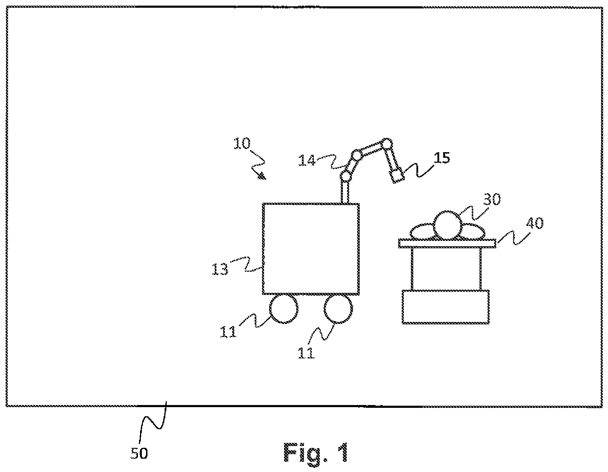

La figure 1 représente schématiquement un robot médical 10 selon l'invention dans une salle 50 d'intervention. La salle 50 d'intervention comporte, de manière conventionnelle, une table 40 sur laquelle est installé un patient 30.

Le robot médical 10 possède une base mobile 13 motorisée permettant au robot médical 10 de se déplacer. Dans l'exemple considéré et illustré à titre d'exemple sur la figure 1, la base mobile 13 du robot médical 10 est équipée de roues 11. Selon d'autres exemples de réalisation, la base mobile 13 du robot médical 10 pourrait être équipée d'autres moyens de déplacement, comme par exemple des chenilles. De préférence, la base mobile 13 est omnidirectionnelle, c'est-à-dire qu'elle permet au robot médical 10 de se déplacer au sol dans toutes les directions par des mouvements de translation et/ou de rotation.

Dans l'exemple considéré et illustré à la figure 1, le robot médical 10 comporte un bras 14 articulé à l'extrémité duquel peut être monté un outil 15. Un tel robot médical 10 peut alors être utilisé pour aider un praticien à positionner, à maintenir, ou à guider l'outil 15.

Le bras 14 articulé comporte de préférence au moins six degrés de liberté pour pouvoir positionner et/ou déplacer l'outil 15 dans l'espace. Rien n'empêche cependant que le bras présente un nombre différent de degrés de liberté.

Par exemple, l'outil 15 peut être un générateur d'ultrasons focalisés à haute intensité qu'il convient de positionner au contact de la peau ou à l'intérieur du patient 30 pour détruire une tumeur.

Selon un autre exemple, l'outil 15 peut être un guide pour implanter un instrument tel qu'un cathéter, une électrode ou une aiguille dans une zone cible du patient 30.

Pour certaines interventions mini-invasives ou percutanées, l'outil 15 peut être un instrument médical, une sonde ou une électrode qu'il convient d'introduire très précisément dans un organe cible pour permettre une biopsie, une résection ou une ablation d'une partie de l'anatomie du patient 30.

Selon encore un autre exemple, l'outil 15 peut être un appareil d'acquisition d'images médicales. Dans des modes particuliers de réalisation, un tel appareil peut être monté sur un support solidaire de la base mobile 13 du robot médical 10, plutôt qu'au bout d'un bras 14 articulé.

Dans la suite de la description, on se place à titre d'exemple et de manière non limitative dans le cas où un outil 15 est monté à l'extrémité d'un bras 14 articulé du robot médical 10.

Il est important que le bras 14 du robot médical 10 puisse positionner l'outil 15 à une position prédéterminée ou déplacer l'outil 15 dans un espace prédéterminé ou selon une trajectoire prédéterminée aussi précisément que possible. Dans ce but, il convient d'identifier une position optimale de la base mobile 13 du robot médical 10 à laquelle le bras 14 articulé du robot médical 10 pourra effectuer les mouvements requis pendant l'intervention.

La base mobile 13 peut avantageusement intégrer un mécanisme d'immobilisation du robot médical 10. Il est alors prévu d'activer ce mécanisme d'immobilisation avant la réalisation de l'intervention médicale, une fois que le robot médical 10 est positionné à une position optimale par rapport à la position du patient 30, afin d'immobiliser la base mobile 13 du robot médical 10 à ladite position optimale. Ceci permet notamment de stabiliser le robot médical 10 lors des mouvements du bras 14 pendant l'intervention.

La qualité de la stabilisation du robot médical 10 est un élément important pour la précision et l'efficacité des actions effectuées par le robot médical 10 pendant une intervention médicale. Il ne s'agit cependant pas d'un élément essentiel pour le positionnement du robot médical 10.

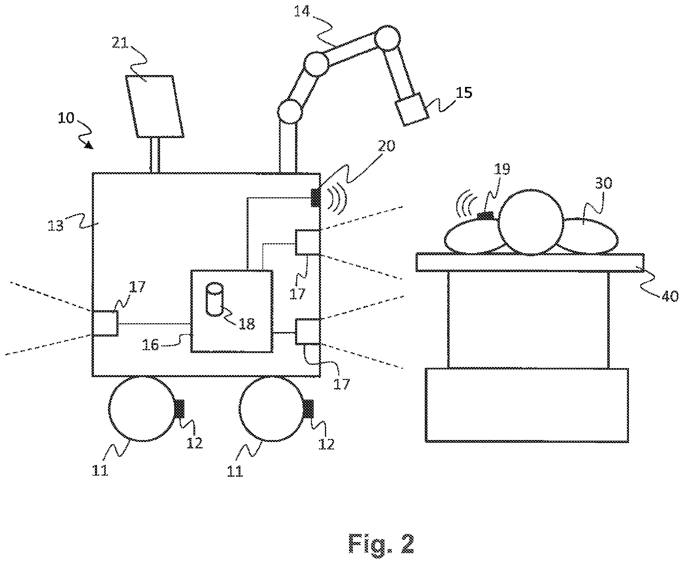

Le mécanisme d'immobilisation peut prendre plusieurs formes. Par exemple, et tel que représenté sur la figure 2, un frein 12 peut être associé à chaque roue 11 de la base mobile 13. Chaque frein 12 peut par exemple être piloté, de manière conventionnelle, par des moyens pneumatiques, hydrauliques ou électriques.

Dans des modes particuliers de réalisation, des stabilisateurs prenant la forme de pieds rétractables associés à des vérins hydrauliques ou électriques pour les abaisser jusqu'au contact avec le sol sont prévus pour supporter tout ou partie du poids du robot médical 10.

Le robot médical 10 est configuré pour identifier de manière autonome, en fonction de la position d'une anatomie d'intérêt du patient 30 et d'un plan d'intervention, au moins une position favorable de la base mobile 13 du robot médical 10 à laquelle le robot médical 10 pourra exécuter les actions prévues pour l'intervention.

Par « anatomie d'intérêt » du patient, on entend une partie au moins du corps humain du patient 30 sur laquelle doit porter l'intervention médicale.

Une position optimale de la base mobile 13 du robot médical 10 pour l'intervention est ensuite sélectionnée parmi la ou les positions favorables identifiées. Le robot médical 10 est configuré pour se déplacer de manière autonome à ladite position optimale.

Il peut être avantageux pour le robot médical 10 de détecter la position de la table 40 et d'identifier la ou les positions favorables en fonction de la position de la table 40. Une table d'intervention est en effet un objet pouvant être reconnu par un algorithme de reconnaissance de formes. En outre, lorsque le patient est installé sur une table 40, ce qui est le cas dans la majorité des cas lorsqu'un patient doit subir une intervention médicale, la détermination d'une position favorable de la base mobile 13 du robot médical 10 doit prendre en compte l'encombrement de la table 40.

Il convient cependant de noter que l'identification de la position d'une table d'intervention n'est pas indispensable au positionnement du robot médical. En effet, dans certains cas le patient n'est pas nécessairement installé sur une table d'intervention, comme par exemple lorsque le robot médical est utilisé pour réaliser des imageries médicales d'un patient qui se tient debout dans une salle d'intervention.

Dans la suite de la description, on considère à titre d'exemple nullement limitatif que le robot médical 10 détecte la position de la table 40 et utilise cette information pour identifier une position favorable de la base mobile 13.

Tel qu'illustré sur la figure 2, le robot médical 10 comporte des capteurs 17 de repérage dans l'espace solidaires de la base mobile 13.

Ces capteurs 17 de repérage dans l'espace peuvent être, par exemple, des caméras : caméras simples, caméras stéréoscopiques, caméras panoramiques, caméras montées sur un mât télescopique, caméras effectuant des rotations à 360°, caméras fonctionnant sur le principe du temps de vol (« Time of Flight » ou ToF dans la littérature anglo-saxonne), caméras de profondeur, dites « RGB-D » (caméras fournissant simultanément une image couleur et une carte de profondeur caractérisant la distance des objets vus dans l'image), etc. Bien entendu, ces différentes caractéristiques de caméras peuvent être combinées entre elles quand cela est techniquement possible (par exemple une caméra RGB-D montée sur un mât télescopique et adaptée pour effectuer une rotation à 360°).

Ces capteurs 17 de repérage dans l'espace peuvent également être des télémètres pour mesurer la distance aux différents éléments de l'environnement : télémètres à ultrason, télémètres à infrarouge, télémètres laser, etc.

Il peut également s'agir de capteurs proprioceptifs permettant de mesurer le déplacement de la base mobile 13 : capteurs odométriques mesurant le déplacement des roues avec un codeur optique, capteurs optiques mesurant le mouvement relatif par rapport au sol, systèmes inertiels avec accéléromètres et gyromètres, etc.

Ces capteurs 17 de repérage dans l'espace sont connus de l'homme du métier. Ils permettent, d'une part, de repérer des objets dans l'espace, par exemple par des méthodes de reconnaissance de formes. Ils permettent, d'autre part, d'estimer les distances séparant différents objets. Ils permettent également au robot médical 10 d'estimer sa position ainsi qu'une distance parcourue lorsque le robot médical 10 se déplace.

Lorsque le robot médical 10 est introduit dans une salle 50 d'intervention, les données fournies par les différents capteurs 17 de repérage dans l'espace permettent notamment de détecter la table 40 d'intervention et le patient 30 installé sur ladite table, et d'estimer les positions de la table 40 et de l'anatomie d'intérêt du patient 30 par rapport au robot médical 10. Les positions de la table 40, du patient 30 et du robot médical 10 peuvent être définies dans un même repère spatial, par exemple un repère en trois dimensions.

Notamment, il est possible d'associer des images provenant d'une caméra RGB-D avec des images médicales par appariement de surface afin de détecter et localiser précisément une anatomie d'intérêt du patient 30. Pour mettre en correspondance les images de la caméra RGB-D avec le référentiel du robot médical 10, il existe au moins deux techniques connues. Une première technique consiste par exemple à s'assurer que la prise de vue de la caméra RGB-D est telle que des éléments géométriques distinctifs du bras 14 du robot médical 10 sont présents sur les images de la caméra RGB-D, afin de localiser à la fois le robot médical 10 et l'anatomie d'intérêt du patient 30. Une deuxième technique consiste à calibrer au préalable la position de la caméra RGB-D sur le robot médical 10.

Dans des modes particuliers de réalisation, un ou plusieurs marqueurs 19 peuvent être positionnés sur l'anatomie d'intérêt du patient 30. Ces marqueurs 19 collaborent avec des capteurs 20 du robot médical 10 afin d'aider à la détection et la localisation de la position de l'anatomie d'intérêt du patient 30. Par exemple, un marqueur 19 lumino-réfléchissant passif ou bien un marqueur 19 actif qui comporte une diode électroluminescente peut être détecté par un capteur 20 spécifique. Selon un autre exemple, un marqueur 19 électromagnétique peut émettre un signal radio qui peut être capté par un capteur 20 embarqué sur le robot médical 10. Le temps mis par un signal radio ou un signal lumineux émis par un capteur 20 et renvoyé par un marqueur 19 peut par exemple permettre de déterminer la distance entre le marqueur 19 et le capteur 20, afin d'en déduire la position et/ou l'orientation de l'anatomie d'intérêt du patient 30. Les capteurs 20 sont portés par le robot médical 10, c'est-à-dire qu'ils sont solidaires de la base mobile 13 du robot médical 10.

Les données fournies par les capteurs 17 de repérage dans l'espace et les capteurs 20 d'aide à la détection de l'anatomie d'intérêt du patient 30 permettent alors de détecter la position du robot médical 10, la position de la table 40 et la position de l'anatomie d'intérêt du patient 30 définies les unes par rapport aux autres dans un même repère spatial.

Ces données sont communiquées à une unité de contrôle 16 du robot médical 10. L'unité de contrôle 16 comporte par exemple un ou plusieurs processeurs et une mémoire 18 (disque dur magnétique, mémoire électronique, disque optique, etc.) dans laquelle est mémorisée un produit programme d'ordinateur, sous la forme d'un ensemble d'instructions de code de programme à exécuter pour mettre en oeuvre les différentes étapes d'un procédé de positionnement automatique du robot médical 10 dans une salle 50 d'intervention. Alternativement ou en complément, le dispositif de contrôle comporte un ou des circuits logiques programmables (FPGA, PLD, etc.), et/ou un ou des circuits intégrés spécialisés (ASIC), et/ou un ensemble de composants électroniques discrets, etc., adaptés à mettre en oeuvre tout ou partie desdites étapes dudit procédé de positionnement.

La communication des données des capteurs 17, 20 du robot médical à l'unité de contrôle 16 peut être assurée, de manière conventionnelle, par des bus informatiques comportant des fils électriques, ou bien par des systèmes de communication sans fil.

L'unité de contrôle 16 est configurée, à l'aide d'algorithmes exploitant les données des capteurs 17, 20 du robot médical 10, pour détecter les positions de la table 40 et de l'anatomie d'intérêt du patient 30 par rapport à la position du robot médical 10, puis pour identifier, à l'aide de ces positions, au moins une position favorable de la base mobile 13 du robot médical 10 pour laquelle le robot médical 10 est capable de dérouler le plan d'intervention.

Une position optimale de la base mobile 13 du robot médical 10 est alors sélectionnée parmi l'ensemble des positions favorables identifiées.

L'unité de contrôle 16 est également configurée pour piloter la base mobile 13 motorisée du robot médical 10. L'unité de contrôle 16 peut alors déplacer le robot médical 10 jusqu'à la position optimale sélectionnée.

Une fois que la base mobile 13 du robot médical 10 a atteint ladite position optimale, l'unité de contrôle 16 peut déclencher un mécanisme d'immobilisation de la base mobile 13 afin d'assurer une stabilisation du robot médical 10 pendant l'intervention.

Selon l'invention, l'unité de contrôle 16 est en outre configurée pour déclencher l'immobilisation de la base mobile 13 du robot médical 10 à la position optimale sélectionnée seulement si un critère de stabilité est vérifié. Dans le cas contraire, l'unité de contrôle 16 est configurée pour déplacer la base mobile du robot médical à une autre position sélectionnée parmi les positions favorables. Ces étapes peuvent être itérées jusqu'à ce qu'une position favorable pour laquelle le critère de stabilité est vérifié soit sélectionnée. Si parmi les positions favorables identifiées, il n'y aucune position pour laquelle le critère de stabilité est vérifiée, alors un message d'alerte peut être émis via une interface utilisateur 21 du robot médical 10.

Le robot médical comporte des pieds rétractables destinés à être abaissés pour immobiliser la base mobile du robot médical. Par exemple, chaque pied comprend une partie suspendue à la base mobile 13 du robot médical 10 ainsi qu'une partie d'appui en relation de coulissement avec la partie suspendue. La partie d'appui comprend une embase du pied destinée à reposer sur la surface du sol lorsque le pied est dans une position d'appui. Lorsque le robot médical 10 n'est pas immobilisé, les pieds occupent une position de retrait et ne sont pas en contact avec la surface du sol.

Selon un premier exemple, chaque pied comporte un capteur permettant de mesurer un défaut local de planéité du sol en regard dudit pied. Pour vérifier le critère de stabilité, l'unité de contrôle 16 peut alors comparer, pour chaque pied, la mesure du défaut local de planéité du sol pour ledit pied avec un seuil prédéterminé. Si le défaut local de planéité du sol pour l'un des pieds est supérieur au seuil, alors le critère de stabilité n'est pas satisfait. Le capteur de mesure d'un défaut local de planéité du sol est adapté pour déterminer s'il existe un décalage entre la position du sol en regard de chaque pied par rapport à une position théorique du sol prédéfinie. Ce décalage correspond à un défaut local de planéité du sol. Le capteur de mesure peut être, par exemple, un capteur à ultrasons dont le fonctionnement en tant que tel est connu de l'homme du métier. Chaque capteur à ultrasons peut être fixé dans l'embase d'un pied du robot médical 10 et dirigé de sorte à mesurer la distance entre ladite embase et la surface du sol à laquelle elle fait face. La mesure est réalisée lorsque le pied est dans une position de retrait pour laquelle la distance entre l'embase du pied et la surface du sol, dans un cas théorique d'absence de défaut local de planéité du sol, est connue.

Selon un deuxième exemple, au niveau de chaque pied un ressort hélicoïdal travaillant en compression est utilisé en combinaison du coulissement de la partie suspendue par rapport à la partie d'appui pour que ledit pied ne supporte qu'une partie du poids du robot médical 10 lorsque ce dernier est immobilisé. Grâce à la mobilité de la partie suspendue relativement à la partie d'appui et au ressort hélicoïdal, chaque pied compense localement d'éventuels défauts locaux de planéité du sol, de type creux ou bosse, de telle sorte que la base mobile 13 du robot médical 10 est supportée de façon sensiblement horizontale et que tous les pieds sont en appui sur le sol malgré d'éventuels défauts locaux de planéité. Cela permet d'assurer la stabilité du robot médical 10. Autrement dit, pour chaque pied, la partie d'appui coulissera plus ou moins le long de la partie suspendue, et le ressort hélicoïdal sera plus ou moins compressé, selon qu'il faut compenser un creux ou une bosse. Chaque pied comporte un capteur permettant de mesurer un poids supporté par ledit pied. Il s'agit par exemple d'un capteur de force permettant de mesurer la compression du ressort hélicoïdal. Pour vérifier le critère de stabilité, l'unité de contrôle 16 peut alors comparer, pour chaque pied, la mesure du poids supporté par ledit pied avec un seuil prédéterminé. Si le poids supporté par l'un des pieds est supérieur à un seuil correspondant au poids maximal qui peut être supporté par chaque pied (ou inférieur à un seuil correspondant à un poids minimal qui doit être supporté par chaque pied), alors le critère de stabilité n'est pas satisfait. Il convient de noter que l'identification des positions favorables de la base mobile 13 du robot médical 10 peut être réalisée après une ou plusieurs étapes de déplacements intermédiaires du robot médical 10. Par exemple, le robot médical 10 peut, dans un premier temps, se rapprocher de la table 40 pour identifier plus précisément la position de l'anatomie d'intérêt du patient 30 avant d'identifier des positions favorables de la base mobile 13 pour l'intervention.

Les positions favorables de la base mobile 13 du robot médical 10 pour l'intervention sont identifiées non seulement en fonction de la position de la table 40 et de la position de l'anatomie d'intérêt du patient 30, mais aussi en fonction d'un plan d'intervention mémorisé dans la mémoire 18 de l'unité de contrôle 16.

Le plan d'intervention comporte une ou plusieurs actions à réaliser sur l'anatomie d'intérêt du patient 30. Une action du plan d'intervention correspond par exemple à pouvoir manipuler l'outil 15 dans un espace prédéterminé, à placer l'outil 15 dans une position prédéterminée, ou bien à déplacer l'outil 15 selon une trajectoire prédéterminée par rapport à l'anatomie d'intérêt du patient 30. Selon un autre exemple, une action du plan d'intervention peut également consister à faire exécuter une opération particulière à l'outil 15, comme par exemple mettre en route un générateur d'ultrasons ou modifier l'intensité d'émission des ultrasons, ou bien déclencher la capture d'une image médicale.

Le plan d'intervention est créé pendant une phase de planification qui précède l'intervention médicale. Lors de cette phase de planification, le praticien définit les différentes actions que le robot médical 10 devra effectuer.

Le plan d'intervention peut par exemple être généré à l'aide d'images médicales de type scanner tomodensitométrie (CT pour « computerized tomography » dans la littérature anglo-saxonne), IRM, PET, ultrasons, rayons X ou autre. Un opérateur, généralement le praticien qui effectuera l'intervention à l'aide du robot médical 10, sélectionne les paramètres de l'outil 15 (par exemple une longueur, un diamètre, une forme 3D, une puissance d'énergie à délivrer, une intensité de courant, un temps de traitement, etc.). Une ou plusieurs trajectoires peuvent être planifiées selon le type de traitement à effectuer pour détruire la tumeur. La planification peut être complètement manuelle, interactive, ou complètement automatisée avec l'aide d'algorithmes de segmentation et de planification. Ces algorithmes d'aide à la décision peuvent être par exemple basés sur des systèmes experts (système capable de reproduire les mécanismes cognitifs du praticien en effectuant un raisonnement à partir de faits et de règles connues) ou sur des mécanismes intelligents d'apprentissage automatique (par exemple avec des réseaux de neurones convolutifs).

Alternativement, ou en complément, la planification de l'intervention peut être réalisée sans images médicales grâce à une représentation en trois dimensions de l'anatomie d'intérêt à traiter. Cette représentation en trois dimensions peut être obtenue par un système d'acquisition d'images non médicales, ou par la collecte de repères ou surfaces anatomiques et l'interpolation d'un modèle statistique ou biomécanique de ces repères ou surfaces. Ce type de planification est particulièrement efficace par exemple dans les interventions sur les structures osseuses comme les articulations (genou, hanche, épaule, etc.).

Les actions du plan d'intervention sont par exemple encodées sous la forme d'instructions connues par l'unité de contrôle 16 dans un fichier informatique. Le fichier informatique correspondant au plan d'intervention peut, par exemple, être généré sur un ordinateur distinct du robot médical 10. Le fichier est alors transmis au robot médical 10 et mémorisé dans la mémoire 18 de l'unité de contrôle 16. Cette transmission du fichier informatique peut être réalisée sous différentes formes, de manière conventionnelle, comme par exemple par une transmission de fichier par clé USB (acronyme anglais de « Universal Serial Bus »), ou bien par une communication sans fil.

Les actions du plan d'intervention décrivent par exemple les différentes positions ou les différents mouvements de l'outil 15 par rapport à l'anatomie d'intérêt du patient 30. L'unité de contrôle 16 connaît en outre le modèle géométrique du bras 14 articulé et de l'outil 15. Par exemple, le bras 14 est équipé de codeurs qui permettent de connaître la position angulaire de chacun de ses axes et, par calcul, de connaître la position de l'outil 15. L'unité de contrôle 16 peut alors déterminer une ou plusieurs positions favorables de la base mobile 13 auxquelles le robot médical 10 sera capable de réaliser les gestes prévus par le plan d'intervention.

Une action du plan d'intervention est une action que le robot médical devra exécuter pendant l'intervention, ultérieurement au procédé de positionnement automatique du robot médical. L'exécution d'une telle action ne fait donc pas partie du procédé de positionnement automatique du robot médical selon l'invention.

Dans un mode particulier de mise en oeuvre, un algorithme d'identification des positions favorables consiste par exemple à discrétiser l'espace des positions possibles du robot médical 10 par rapport à l'anatomie d'intérêt du patient 30 et à définir un paramètre d'accessibilité représentatif d'un nombre de configurations possibles du bras 14 articulé permettant d'effectuer une action du plan d'intervention lorsque la base mobile 13 du robot médical 10 est positionnée à une position donnée. Le bras 14 articulé comporte une ou plusieurs articulations contrôlées par l'unité de contrôle 16. Une configuration possible du bras articulé correspond alors à un ensemble de valeurs de paramètres prises par la ou les articulations du bras (par exemple un angle de rotation, une distance de translation, etc.).

On entend par « position possible » une position de la base mobile 13 du robot médical 10 à laquelle le bras 14 articulé peut atteindre l'anatomie d'intérêt du patient. L'ensemble des positions possibles correspond ainsi à un espace travail dans lequel l'anatomie d'intérêt du patient est à portée du bras 14 articulé.

Pour un bras 14 articulé du robot médical 10 ayant plus de degrés de liberté que nécessaire pour effectuer une action planifiée, plusieurs configurations du bras 14 peuvent être envisagées. Par exemple, le bras 14 articulé d'un robot médical 10 anthropomorphe à six degrés de liberté peut généralement positionner un guide-aiguille selon une direction rectiligne passant par un point d'entrée prédéterminé selon plusieurs configurations différentes de ses axes. Si une seule configuration est possible, le paramètre d'accessibilité sera moindre que si deux configurations différentes sont possibles.

Dans l'exemple considéré, une position favorable correspond à une position de la base mobile 13 du robot médical 10 à laquelle il existe, pour chaque action du plan d'intervention, au moins une configuration du bras 14 articulé permettant d'effectuer ladite action.

Il convient de noter la différence entre « position possible » et « position favorable » : si l'anatomie d'intérêt est accessible par le bras 14 articulé à une position donnée de la base mobile 13, cela ne signifie pas nécessairement qu'il existe une configuration du bras 14 articulé, lorsque la base mobile 13 est positionnée à cette position, pour réaliser chaque action du plan d'intervention.

Le paramètre d'accessibilité peut également être défini en fonction de valeurs représentatives des débattements disponibles des articulations du bras 14 pour les différentes configurations possibles dudit bras 14 permettant d'effectuer une action du plan d'intervention à une position favorable donnée. Le débattement d'une articulation est par exemple limité par une ou plusieurs butées. On entend par « débattement disponible » d'une articulation un paramètre représentatif de l'amplitude d'un mouvement permis par ladite articulation, comme par exemple un angle entre la position d'un segment de l'articulation pour une configuration donnée et la butée la plus proche.

Pour chaque position favorable identifiée, un niveau global d'accessibilité peut alors être calculé en fonction des paramètres d'accessibilité de toutes les actions du plan d'intervention. Par exemple, un niveau global d'accessibilité pour une position favorable identifiée peut correspondre à la somme des paramètres d'accessibilité de toutes les actions du plan d'intervention lorsque la base mobile 13 du robot médical 10 est positionnée à ladite position favorable. Il existe bien entendu d'autres méthodes pour calculer un niveau global d'accessibilité. Par exemple, des pondérations peuvent être utilisées en fonction de l'importance d'une action relativement à d'autres actions du plan d'intervention.

L'unité de contrôle 16 peut alors être configurée pour sélectionner une position optimale parmi l'ensemble des positions favorables identifiées en fonction des paramètres d'accessibilité ou du niveau global d'accessibilité calculés pour les différentes positions favorables identifiées. Par exemple, la position optimale peut correspondre à la position favorable pour laquelle le paramètre d'accessibilité d'une action particulière du plan d'intervention a la plus grande valeur. Selon un autre exemple, la position optimale peut correspondre à la position favorable pour laquelle le niveau global d'accessibilité est le plus important.

De telles dispositions permettent une plus grande flexibilité pendant l'intervention. En effet, le praticien pourra potentiellement choisir, une fois le robot positionné à la position optimale, pendant l'intervention, entre plusieurs configurations possibles pour effectuer telle ou telle action du plan d'intervention. La préférence d'une configuration particulière par rapport à une autre pourra dépendre du déroulement de l'intervention.

Dans des modes particuliers de mise en oeuvre, l'unité de contrôle 16 met en oeuvre des algorithmes de calcul de la position optimale prenant en compte des informations d'une base de données d'interventions médicales déjà réalisées : position réelle du robot médical 10 par rapport à l'anatomie d'intérêt du patient 30, paramètres d'accessibilité des actions planifiées et réalisées, positions et mouvements de l'outil 15 inaccessibles pendant l'intervention, etc.

Ces algorithmes peuvent se baser sur des méthodes d'apprentissage automatique connues (apprentissage par réseaux de neurones, apprentissage automatique supervisé, semi-supervisé ou non supervisé, etc.). Ces algorithmes sont avantageux car au lieu de proposer des positions optimales théoriques, ils peuvent proposer des positions optimales prenant en compte la réalité d'interventions déjà réalisées, ces dernières positions pouvant être significativement différentes des premières en fonction de la complexité de la modélisation théorique du problème de positionnement de la base mobile 13 par rapport à l'anatomie d'intérêt du patient 30. L'unité de contrôle 16 peut être configurée pour mémoriser, pendant une intervention médicale, des informations sur la position du robot médical 10 par rapport à l'anatomie d'intérêt du patient 30, ainsi que sur des paramètres d'accessibilité, de sorte que ces données soient réutilisées ultérieurement par des algorithmes d'intelligence artificielle pour optimiser le positionnement du robot médical 10 lors d'une intervention future.

Des données d'entraînement, correspondant aux informations relatives aux interventions déjà réalisées, telles que les actions planifiées qui nécessitent des positions de l'outil 15 par rapport au patient 30, la position réelle de la base mobile 13 du robot médical 10 par rapport au patient 30 lors de l'intervention, les positions atteintes, les positions inaccessibles, les débattements disponibles et autres mouvements, sont par exemple mémorisés dans la mémoire 18 de l'unité de contrôle 16. Un processeur de l'unité de contrôle 16 peut alors créer un modèle profond de réseau de neurones multicouche à partir des données d'entrainement. Ce modèle est mémorisé dans la mémoire 18 de l'unité de contrôle 16. Lors de nouvelles interventions, le modèle de réseau de neurones entrainé est appliqué aux actions planifiées et participe à déterminer la position optimale pour réaliser le plan d'intervention.

Dans des modes particuliers de mise en oeuvre, des préférences d'un praticien peuvent être mémorisées dans la mémoire 18 de l'unité de contrôle 16 et prises en compte lors de la sélection de la position optimale du robot médical 10. Les préférences peuvent tenir compte, par exemple, de la position prévue du patient 30 sur la table 40 de la salle 50 d'intervention (décubitus dorsal, ventral, latéral), de la latéralité (droitier ou gaucher) du praticien, d'un côté préféré de la table 40 pour installer le robot, etc.

Dans des modes particuliers de réalisation, le robot médical 10 comporte une interface utilisateur 21 permettant au robot médical 10 de présenter une information au praticien et d'indiquer une commande au robot médical 10. Cette interface utilisateur 21 comporte par exemple un écran, un clavier, une souris, un bouton, un joystick, un écran tactile, un système de reconnaissance vocale ou de gestes, ou tout autre moyen permettant au robot médical 10 de présenter une information au praticien et de recevoir de la part du praticien un signal associée à une commande connue. Par exemple, plutôt que de sélectionner de manière autonome la position optimale, le robot médical 10 peut présenter l'ensemble des positions favorables identifiées sur un écran et laisser au praticien le choix de la position optimale parmi l'ensemble de ces positions favorables. Selon un autre exemple, pendant l'intervention, si plusieurs configurations du bras 14 articulé sont possibles pour effectuer une action du plan d'intervention, ces différentes configurations peuvent être présentées sur un écran afin que le praticien choisisse la configuration préférée.

Dans des modes particuliers de mise en oeuvre, l'interface utilisateur 21 fournit une indication au praticien que le robot médical 10 a atteint la position optimale, et le praticien peut valider cette position et déclencher l'immobilisation de la base mobile 13 à ladite position optimale.

Dans une variante, l'unité de contrôle 16 déclenche de façon autonome l'immobilisation de la base mobile 13 lorsque le robot médical 10 a atteint la position optimale.

Dans des modes particuliers de mise en oeuvre, le robot médical 10 génère une cartographie de la salle 50 d'intervention pour déterminer la position optimale à atteindre.

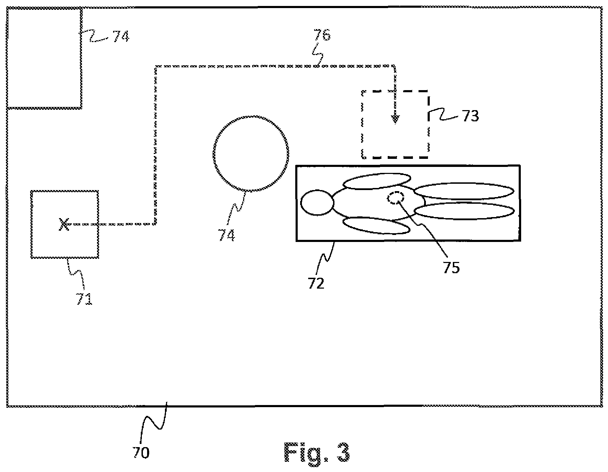

La figure 3 représente schématiquement une cartographie 70 de la salle 50 d'intervention et un trajet 76 du robot médical 10 pour atteindre une position optimale 73 pour traiter le patient 30.

Il convient de noter que la figure 3 représente schématiquement une cartographie 70 en deux dimensions, avec une vue de dessus, mais rien n'empêche que ladite cartographie 70 soit réalisée en trois dimensions. Une cartographie en trois dimensions permet avantageusement de mieux prendre en compte l'encombrement des différents éléments présents dans la salle 40 d'intervention.

Pendant le procédé de positionnement automatique du robot médical 10, une fois que le patient 30 a été installé dans la salle 50 d'intervention, la cartographie 70 est par exemple réalisée par l'unité de contrôle 16 du robot médical 10. La cartographie 70 est par exemple réalisée à l'aide d'un capteur 17 de repérage dans l'espace de type caméra RGB-D montée sur un mât télescopique fixé à la base mobile 13, et à l'aide d'un algorithme de reconnaissance de formes. L'unité de contrôle 16 détermine alors, sur la cartographie 70, la position 71 du robot, la position 72 de la table 40, la position 75 de l'anatomie d'intérêt à traiter, ainsi que les positions 74 d'éventuels obstacles pouvant entraver les déplacements du robot médical 10.

L'unité de contrôle 16 est configurée pour identifier, en fonction de la position 71 du robot médical 10, de la position 72 de la table 40, de la position 75 de l'anatomie d'intérêt du patient 30 et du plan d'intervention mémorisé, au moins une position favorable à laquelle le plan d'intervention peut être déroulé par le robot médical 10. Une position optimale 73 est alors sélectionnée, et un trajet 76 est déterminé par l'unité de contrôle 16 pour que le robot médical 10 puisse atteindre la position optimale 73 en tenant compte des positions 74 d'éventuels obstacles pour les éviter.

Il convient de noter que des déplacements intermédiaires peuvent être effectués par le robot médical 10, par exemple pour se rapprocher de la table 40, avant de déterminer précisément la position optimale 73.

La base mobile 13 peut intégrer des pare-chocs pour minimiser les dégâts en cas de choc. Ces pare-chocs peuvent être combinés avec des capteurs tactiles permettant d'arrêter automatiquement le mouvement de la base mobile 13 en cas de contact avec un obstacle. Alternativement, ou en complément, le déplacement du robot médical 10 peut être arrêté manuellement, par exemple avec un bouton d'urgence.

L'assistance motorisée de la base mobile 13 et les capteurs 17 de repérage dans l'espace du robot médical 10 peuvent également être mis en oeuvre dans le cadre d'un procédé de transfert du robot médical 10 depuis une zone de stockage du robot médical 10 jusqu'à la salle 50 d'intervention, et inversement. Un tel procédé de transfert du robot médical 10 est mis en oeuvre avant et/ou après le procédé de positionnement automatique du robot médical.

Le transfert du robot médical 10 depuis sa zone de stockage jusqu'à la salle 50 d'intervention peut être réalisé de façon autonome par le robot médical 10 ou bien avec l'assistance d'un opérateur.

Lorsque le robot médical 10 se trouve dans sa zone de stockage, il peut se rendre dans la salle 50 d'intervention en suivant un opérateur situé à l'avant ou à l'arrière de la base mobile 13 à l'aide des capteurs 17 de repérage dans l'espace et de moyens de conduite mis en oeuvre par l'unité de contrôle 16 (module de détection de position relative de l'opérateur, module de consigne de déplacement, module de guidage, module de détection d'obstacles, etc.).

La base mobile 13 peut aussi intégrer une ou plusieurs caméras à l'avant et sur les côtés afin de retransmettre sur un écran des images de l'environnement autour du robot médical 10 permettant à l'opérateur situé à l'arrière de la base mobile 13 de mieux se repérer et faciliter le déplacement dans un couloir, le passage d'une porte, ou l'évitement d'obstacles.

Lorsque la base mobile 13 se trouve dans sa zone de stockage, elle peut se rendre dans une salle 50 d'intervention de façon entièrement autonome lorsque les différentes pièces du bâtiment dans lequel elle se trouve ont été cartographiées au préalable. Dans ce cas, lors d'une étape préalable, les informations concernant l'emplacement de la zone de stockage et celui de la salle 50 d'intervention sont indiquées au robot médical 10. Les portes d'accès à ces différentes zones sont par exemple munies de capteurs ou de codes QR (abréviation de l'anglais « Quick Response ») permettant au robot médical 10 de détecter lesdites portes d'accès et auxdites portes d'accès d'autoriser le passage du robot médical 10.

Une fois que le robot médical 10 se trouve dans la salle 50 d'intervention, il peut alors se positionner automatiquement à une position optimale 73 pour l'intervention.

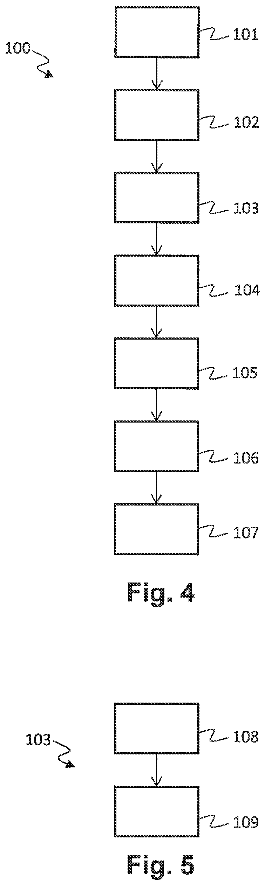

La figure 4 représente schématiquement les principales étapes d'un procédé 100 de positionnement automatique d'un robot médical 10 selon l'invention tel que décrit en référence aux figures 1 à 3.

Le procédé 100 de positionnement automatique est mis en oeuvre par l'unité de contrôle 16 du robot médical 10.

Le procédé 100 de positionnement automatique du robot médical 10 commence lorsqu'il pénètre dans la salle 50 d'intervention au début de l'intervention médicale. Le patient 30 est alors installé sur une table 40 dans la salle 50 d'intervention.

Le procédé 100 comporte une étape de détection 101 de la position 75 de l'anatomie d'intérêt du patient 30 par rapport à une position 71 du robot médical 10. Dans l'exemple considéré, la position 72 la table 40 est également détectée dans cette étape. Cette étape est réalisée de façon autonome par le robot médical 10.

Le procédé 100 comporte également une étape d'identification 102, à partir de la position 75 de l'anatomie d'intérêt du patient 30 et du plan d'intervention, d'au moins une position favorable de la base mobile 13 du robot médical 10 à laquelle le robot médical 10 est capable d'effectuer les actions du plan d'intervention. Dans l'exemple considéré, cette étape identification 102 est réalisée en outre à partir de la position 72 de la table 40. Cette étape est réalisée de façon autonome par le robot médical 10.

Une sélection 103 d'une position optimale 73 parmi l'ensemble des positions favorables est alors effectuée. Comme expliqué précédemment, la sélection 103 de la position optimale 73 peut être faite en fonction des paramètres d'accessibilité ou d'un niveau global d'accessibilité calculé pour chaque position favorable, en fonction de préférences mémorisées, et/ou à l'aide d'informations extraites d'une base de données d'interventions médicales déjà réalisées. Cette sélection 103 peut éventuellement être réalisée par le praticien à qui les différentes positions favorables sont présentées sur une interface utilisateur 21.

De manière optionnelle, le procédé 100 de positionnement peut comporter une génération 104 d'une cartographie 70 de la salle 50 d'intervention sur laquelle est représentée une position 71 du robot médical 10, la position 72 de la table 40, la position 75 de l'anatomie d'intérêt du patient 30, et la position optimale 73 à atteindre, ainsi qu'une détermination 105, à partir de ladite cartographie 70, d'un trajet 76 du robot médical 10 pour atteindre ladite position optimale 73.

Le procédé 100 comporte une étape de déplacement 106 du robot médical 10 à la position optimale 73. Ce déplacement 106 est réalisé de façon autonome par le robot médical 10.

Il convient de noter que l'ordre des étapes citées ci-avant n'est pas nécessairement figé. Aussi, une ou plusieurs étapes de déplacement intermédiaires du robot médical 10 en direction de la table 40 peuvent avoir lieu avant que le robot médical 10 ne soit en mesure de détecter l'anatomie d'intérêt du patient 30 et d'identifier des positions favorables pour réaliser l'intervention.

Il est également important de noter qu'aucune cartographie n'a besoin d'être fournie au robot médical 10. Ainsi, le robot médical 10 peut être initialement dépourvu d'une cartographie de la salle 50 d'intervention au début du procédé 100 de positionnement automatique.

Le procédé de positionnement automatique du robot médical 10 peut avantageusement comporter une étape d'immobilisation 107 de la base mobile 13 lorsque la position optimale 73 a été atteinte. Cette étape d'immobilisation 107 peut être réalisée de manière autonome par le robot médical 10, ou bien avec la participation d'un opérateur. Par exemple, l'immobilisation 107 peut être déclenchée par une commande du praticien après validation de la position optimale atteinte sur l'interface utilisateur 21.

Comme expliqué précédemment, l'immobilisation de la base mobile du robot médical à la position optimale peut n'être déclenchée que si un critère de stabilité de la base mobile du robot médical est vérifié. Dans le cas contraire, le procédé de positionnement automatique selon l'invention peut comporter une étape supplémentaire de déplacement de la base mobile du robot médical à une autre position sélectionnée parmi les positions favorables.

Dans l'exemple considéré, le robot médical 10 comporte un bras 14 articulé et un outil 15 monté à une extrémité dudit bras 14. Le bras 14 est configuré par l'unité de contrôle 16 du robot médical 10 pour effectuer une action du plan d'intervention. Tel qu'illustré à la figure 5, la sélection 103 de la position optimale 73 peut alors comporter :
- un calcul 108, pour chaque action du plan d'intervention et pour chaque position favorable identifiée, d'un paramètre d'accessibilité en fonction du nombre de configurations possibles du bras 14 articulé pour effectuer ladite action lorsque la base mobile 13 du robot médical 10 est positionnée à ladite position favorable,
- un calcul 109, pour chaque position favorable identifiée, d'un niveau global d'accessibilité du plan d'intervention, ledit niveau global d'accessibilité étant calculé en fonction des paramètres d'accessibilité de toutes les actions du plan d'intervention lorsque la base mobile 13 du robot médical 10 est positionnée à ladite position favorable.

Il convient de noter que le calcul 108 du paramètre d'accessibilité peut également être fait en fonction de valeurs représentatives des débattements disponibles des articulations du bras 14 pour les différentes configurations possibles du bras 14 permettant d'effectuer une action du plan d'intervention à une position favorable donnée.

La description ci-avant illustre clairement que, par ses différentes caractéristiques et leurs avantages, la présente invention atteint les objectifs fixés. En particulier, elle propose une solution pour faciliter et fiabiliser le positionnement automatique d'un robot médical 10 dans une salle 50 d'intervention.

Les étapes du procédé 100 de positionnement selon l'invention sont majoritairement mises en oeuvre de façon autonome par le robot médial 10, ce qui supprime les erreurs de positionnement liées à l'intervention d'un opérateur ou d'un dispositif supplémentaire utilisé pour diriger le robot. En outre, l'absence d'un dispositif supplémentaire distinct du robot médical 10 limite les efforts de maintenance d'un système d'assistance robotisée à une intervention médicale.

En outre, le robot médical 10 selon l'invention est capable de s'adapter à la position réelle de l'anatomie d'intérêt du patient 30 au moment de l'intervention, celle-ci pouvant être significativement différente d'une anatomie modélisée pendant une phase de planification.

De plus, le robot médical 10 est capable de se positionner automatiquement dans n'importe quelle salle 50 d'intervention, même si le robot médical 10 n'a pas de cartographie ou d'informations spécifiques relatives à l'agencement de la salle 50 avant le début du procédé de positionnement automatique du robot médical 10. Cela limite alors les coûts et les efforts de maintenance relatifs aux interventions médicales assistées par un robot médical.

## Revendications

1. Procédé (100) de positionnement automatique d'un robot médical pour réaliser une intervention médicale sur un patient (30), ledit robot médical (10) comportant une base mobile (13) motorisée permettant de déplacer le robot médical (10), et une unité de contrôle (16) mémorisant un plan d'intervention comportant au moins une action à effectuer sur une anatomie d'intérêt dudit patient (30), ledit procédé (100) de positionnement comportant les étapes suivantes, mises en oeuvre de manière autonome par le robot médical (10) :
- une détection (101) de la position de l'anatomie d'intérêt du patient (30) par rapport à une position du robot médical (10),
- une identification (102), à partir de la position de l'anatomie d'intérêt du patient (30) et du plan d'intervention, d'au moins deux positions favorables différentes de la base mobile (13) du robot médical (10) auxquelles le robot médical (10) est capable d'effectuer ladite au moins une action du plan d'intervention,
- un déplacement (106) de la base mobile (13) du robot médical (10) à une position optimale sélectionnée parmi les positions favorables identifiées,
- une vérification d'un critère de stabilité de la base mobile (13) du robot médical (10) à la position optimale sélectionnée,
- si ladite vérification est positive, une immobilisation de la base mobile (13) du robot médical (10) à ladite position optimale,
- si ladite vérification est négative, un déplacement de la base mobile (13) du robot médical (10) à une autre position sélectionnée parmi les positions favorables,
**caractérisé en ce que** le robot médical (10) comporte des pieds rétractables destinés à être abaissés pour immobiliser la base mobile (13) du robot médical (10), chaque pied comportant un capteur permettant de mesurer un poids supporté par ledit pied, et la vérification d'un critère de stabilité comporte, pour chaque pied, une comparaison de la mesure du poids supporté par ledit pied avec un seuil prédéterminé.

2. Procédé (100) selon la revendication 1 dans lequel le patient (30) est installé sur une table (40) d'une salle (50) d'intervention, le procédé (100) comporte une détection de la position de la table (40), l'identification (102) des positions favorables de la base mobile (13) du robot médical (10) est effectuée en outre à partir de la position de la table (40), et la détection de la position de la table (40) et la détection de l'anatomie d'intérêt du patient (30) sont effectuées à partir d'informations provenant de capteurs (17) de repérage dans l'espace portés par le robot médical (10).

3. Procédé (100) selon la revendication 2 dans laquelle le robot est initialement dépourvu d'une cartographie de la salle (50) d'intervention et le procédé (100) comporte en outre les étapes suivantes mises en oeuvre de manière autonome par le robot médical (10) :
- une génération (104) d'une cartographie (70) de la salle (50) d'intervention sur laquelle sont représentées une position (71) du robot médical (10), la position (72) de la table (40), la position (75) de l'anatomie d'intérêt du patient (30), et la position optimale (73),
- une détermination (105), à partir de ladite cartographie (70), d'un trajet (76) du robot médical (10) pour atteindre ladite position optimale (73).

4. Procédé (100) selon l'une des revendications 1 à 3 dans lequel :
- le robot médical (10) comporte un bras (14) articulé et un outil (15) monté à une extrémité dudit bras (14), une ou plusieurs articulations dudit bras (14) étant contrôlées par l'unité de contrôle (16) du robot médical (10) pour positionner le bras(14) selon une configuration possible,
- une position favorable correspond à une position de la base mobile (13) du robot médical (10) à laquelle il existe, pour chaque action du plan d'intervention, au moins une configuration possible du bras (14) articulé permettant d'effectuer ladite action,
- le procédé (100) comporte un calcul (108), pour chaque action du plan d'intervention et pour chaque position favorable identifiée, d'un paramètre d'accessibilité en fonction d'un nombre de configurations possibles du bras (14) articulé pour effectuer ladite action lorsque la base mobile (13) du robot médical (10) est positionnée à ladite position favorable,
- la position optimale (73) est sélectionnée en fonction des paramètres d'accessibilité des positions favorables identifiées.

5. Procédé (100) selon la revendication 4 dans lequel ledit paramètre d'accessibilité est calculé en outre en fonction de débattements disponibles des articulations du bras (14) pour chacune desdites configurations possibles du bras (14).

6. Procédé (100) selon l'une des revendications 4 à 5 dans lequel :
- le procédé (100) comporte en outre un calcul (109), pour chaque position favorable identifiée, d'un niveau global d'accessibilité du plan d'intervention, ledit niveau global d'accessibilité étant calculé en fonction des paramètres d'accessibilité de chaque action du plan d'intervention lorsque la base mobile (13) du robot médical (10) est positionnée à ladite position favorable,
- la position optimale (73) est sélectionnée en fonction des niveaux globaux d'accessibilité calculés pour les positions favorables identifiées.

7. Procédé (100) selon l'une des revendications 4 à 6 dans lequel une action du plan d'intervention correspond à permettre le déplacement de l'outil (15) dans un volume prédéterminé par rapport à ladite anatomie d'intérêt du patient (30), ou à permettre le déplacement de l'outil (15) selon une trajectoire prédéterminée par rapport à ladite anatomie d'intérêt du patient (30), ou à placer l'outil (15) dans une position prédéterminée par rapport à ladite anatomie d'intérêt du patient (30).

8. Procédé (100) selon l'une des revendications 1 à 7 dans lequel la position optimale (73) est sélectionnée en fonction de préférences d'un opérateur mémorisées dans l'unité de contrôle (16).

9. Procédé (100) selon l'une des revendications 1 à 8 dans lequel la position optimale (73) est sélectionnée par un algorithme d'apprentissage automatique en fonction d'informations extraites d'une base de données d'interventions médicales déjà réalisées.

10. Produit programme d'ordinateur **caractérisé en ce qu'**il comporte un ensemble d'instructions de code de programme qui, lorsqu'elles sont exécutées par un ou plusieurs processeurs, configurent le ou les processeurs pour mettre en oeuvre un procédé (100) de positionnement automatique d'un robot médical selon l'une des revendications 1 à 9.

11. Robot médical (10) comportant une base mobile (13) motorisée, des capteurs (17) de repérage dans l'espace solidaires de la base mobile (13), et une unité de contrôle (16) mémorisant un plan d'intervention comportant au moins une action à réaliser sur une anatomie d'intérêt d'un patient (30), ladite unité de contrôle (16) étant configurée pour :
- détecter, à partir d'informations provenant des capteurs (17) de repérage dans l'espace, une position de l'anatomie d'intérêt du patient (30) par rapport au robot médical (10),
- identifier, à partir de la position de l'anatomie d'intérêt du patient (30) et du plan d'intervention, au moins deux positions favorables de la base mobile (13) du robot médical (10) auxquelles le robot médical (10) est capable d'effectuer ladite au moins une action du plan d'intervention,
- déplacer la base mobile (13) du robot médical (10) à une position optimale sélectionnée parmi les positions favorables identifiées,
- vérifier un critère de stabilité de la base mobile (13) du robot médical (10) à la position optimale sélectionnée,
- si ladite vérification est positive, déclencher une immobilisation de la base mobile du robot médical (10) à ladite position optimale,
- si ladite vérification est négative, déplacer la base mobile (13) du robot médical (10) à une autre position sélectionnée parmi les positions favorables,
**caractérisé en ce que** le robot médical (10) comporte des pieds rétractables destinés à être abaissés pour immobiliser la base mobile (13) du robot médical (10), chaque pied comportant un capteur permettant de mesurer un poids supporté par ledit pied, et pour vérifier le critère de stabilité, l'unité de contrôle (16) est configurée pour comparer, pour chaque pied, la mesure du poids supporté par ledit pied avec un seuil prédéterminé.

12. Robot médical (10) selon la revendication 11 dans lequel l'unité de contrôle (16) est également configurée pour détecter, à partir d'informations provenant des capteurs (17) de repérage dans l'espace, une position d'une table (40) d'une salle (50) d'intervention sur laquelle est installé le patient (30), et pour identifier ladite au moins une position favorable en fonction de la position de la table (40).

13. Robot médical (10) selon la revendication 12 dans lequel l'unité de contrôle (16) est également configurée pour :
- générer une cartographie (70) de la salle (50) d'intervention sur laquelle sont représentées une position (71) du robot médical (10), la position (72) de la table (40), la position (75) de l'anatomie d'intérêt du patient (30), et la position optimale (73),
- déterminer, à partir de ladite cartographie (70), un trajet (76) du robot médical (10) pour atteindre ladite position optimale (73).

14. Robot médical (10) selon l'une des revendications 11 à 13 dans lequel le robot médical (10) comporte en outre des capteurs (20) d'aide à la détection de l'anatomie d'intérêt du patient (30) coopérant avec des marqueurs (19) positionnés sur ladite anatomie d'intérêt, les capteurs (20) d'aide à la détection de l'anatomie d'intérêt du patient (30) étant solidaires de la base mobile (13) du robot médical (10), et dans lequel les informations provenant de capteurs utilisées pour identifier la ou les positions favorables sont fournies exclusivement par des capteurs portés par le robot médical.

15. Robot médical (10) selon l'une des revendications 11 à 14 comportant un bras (14) articulé et un outil (15) monté à une extrémité dudit bras (14), une ou plusieurs articulations dudit bras (14) étant contrôlées par l'unité de contrôle (16) du robot médical (10) pour positionner le bras(14) selon une configuration possible, une position favorable correspondant à une position de la base mobile (13) du robot médical (10) à laquelle il existe, pour chaque action du plan d'intervention, au moins une configuration possible du bras (14) articulé permettant d'effectuer ladite action, et l'unité de contrôle (16) est en outre configurée pour :
- calculer, pour chaque action du plan d'intervention et pour chaque position favorable identifiée, un paramètre d'accessibilité représentatif d'un nombre de configurations possibles du bras (14) articulé pour effectuer ladite action lorsque la base mobile (13) du robot médical (10) est positionnée à ladite position favorable,
- sélectionner la position optimale en fonction des paramètres d'accessibilité des positions favorables identifiées.

## Patentansprüche

1. Verfahren (100) zur automatischen Positionierung eines medizinischen Roboters zur Durchführung eines medizinischen Eingriffs an einem Patienten (30), wobei der medizinische Roboter (10) einen motorisierten beweglichen Sockel (13), der es ermöglicht, den medizinischen Roboter (10) zu bewegen, und eine Steuereinheit (16) aufweist, die einen Eingriffsplan speichert, der wenigstens eine Aktion aufweist, die an einer Anatomie von Interesse des Patienten (30) durchzuführen ist, wobei das Positionierungsverfahren (100) die folgenden Schritte aufweist, die vom medizinischen Roboter (10) eigenständig durchgeführt werden:
- ein Detektieren (101) der Position der Anatomie von Interesse des Patienten (30) im Verhältnis zu einer Position des medizinischen Roboters (10),
- ein Ermitteln (102), anhand der Position der Anatomie von Interesse des Patienten (30) und des Eingriffsplans, wenigstens zweier verschiedener günstiger Positionen des beweglichen Sockels (13) des medizinischen Roboters (10), in denen der medizinische Roboter (10) imstande ist, die wenigstens eine Aktion des Eingriffsplans durchzuführen,
- ein Bewegen (106) des beweglichen Sockels (13) des medizinischen Roboters (10) in eine aus den ermittelten günstigen Positionen ausgewählte optimale Position,
- ein Überprüfen eines Stabilitätskriteriums des beweglichen Sockels (13) des medizinischen Roboters (10) in der ausgewählten optimalen Position,
- wenn die Überprüfung positiv ist, ein Festsetzen des beweglichen Sockels (13) des medizinischen Roboters (10) in der optimalen Position,
- wenn die Überprüfung negativ ist, ein Bewegen des beweglichen Sockels (13) des medizinischen Roboters (10) in eine aus den günstigen Positionen ausgewählte andere Position,
**dadurch gekennzeichnet, dass** der medizinische Roboter (10) einziehbare Füße aufweist, die dazu bestimmt sind, abgesenkt zu werden, um den beweglichen Sockel (13) des medizinischen Roboters (10) festzusetzen, wobei jeder Fuß einen Sensor aufweist, mit dem ein Gewicht gemessen werden kann, das von dem Fuß getragen wird, und das Überprüfen eines Stabilitätskriteriums für jeden Fuß ein Vergleichen der Messung des Gewichts, das von dem Fuß getragen wird, mit einer vorbestimmten Schwelle aufweist.

2. Verfahren (100) nach Anspruch 1, wobei der Patient (30) auf einem Tisch (40) eines Behandlungsraumes (50) gelagert ist, das Verfahren (100) ein Detektieren der Position des Tisches (40) aufweist, das Ermitteln (102) der günstigen Positionen des beweglichen Sockels (13) des medizinischen Roboters (10) ferner anhand der Position des Tisches (40) durchgeführt wird und das Detektieren der Position des Tisches (40) und das Detektieren der Anatomie von Interesse des Patienten (30) anhand von Informationen durchgeführt werden, die von Sensoren (17) zur Positionsbestimmung im Raum stammen, die von dem medizinischen Roboter (10) getragen werden.

3. Verfahren (100) nach Anspruch 2, wobei der Roboter anfangs keine Kartografie des Behandlungsraumes (50) aufweist und das Verfahren (100) ferner die folgenden Schritte aufweist, die vom medizinischen Roboter (10) eigenständig durchgeführt werden:
- ein Erzeugen (104) einer Kartografie (70) des Behandlungsraumes (50), auf der eine Position (71) des medizinischen Roboters (10), die Position (72) des Tisches (40), die Position (75) der Anatomie von Interesse des Patienten (30) und die optimale Position (73) dargestellt sind,
- ein Bestimmen (105), anhand der Kartografie (70), eines Wegs (76) des medizinischen Roboters (10) zur Erreichung der optimalen Position (73).

4. Verfahren (100) nach einem der Ansprüche 1 bis 3, wobei:
der medizinische Roboter (10) einen Gelenkarm (14) und ein Werkzeug (15) aufweist, das an einem Ende des Arms (14) angebracht ist, wobei ein oder mehrere Gelenke des Arms (14) von der Steuereinheit (16) des medizinischen Roboters (10) gesteuert werden, um den Arm (14) entsprechend einer möglichen Konfiguration zu positionieren,
- eine günstige Position einer Position des beweglichen Sockels (13) des medizinischen Roboters (10) entspricht, in der für jede Aktion des Eingriffsplans wenigstens eine mögliche Konfiguration des Gelenkarms (14) vorhanden ist, mit der die Aktion durchgeführt werden kann,
- das Verfahren (100) ein Berechnen (108), für jede Aktion des Eingriffsplans und für jede ermittelte günstige Position, eines Erreichbarkeitsparameters je nach einer Anzahl von möglichen Konfigurationen des Gelenkarms (14) aufweist, um die Aktion durchzuführen, wenn der bewegliche Sockel (13) des medizinischen Roboters (10) in der günstigen Position positioniert ist,
- die optimale Position (73) je nach den Erreichbarkeitsparametern der ermittelten günstigen Positionen ausgewählt wird.

5. Verfahren (100) nach Anspruch 4, wobei der Erreichbarkeitsparameter ferner je nach verfügbaren Auslenkungen der Gelenke des Arms (14) für jede der möglichen Konfigurationen des Arms (14) berechnet wird.

6. Verfahren (100) nach einem der Ansprüche 4 bis 5, wobei:
- das Verfahren (100) ferner ein Berechnen (109), für jede ermittelte günstige Position, eines Gesamterreichbarkeitsniveaus des Eingriffsplans aufweist, wobei das Gesamterreichbarkeitsniveau je nach den Erreichbarkeitsparametern jeder Aktion des Eingriffsplans berechnet wird, wenn der bewegliche Sockel (13) des medizinischen Roboters (10) in der günstigen Position positioniert ist,
- die optimale Position (73) je nach den Gesamterreichbarkeitsniveaus ausgewählt wird, die für die ermittelten günstigen Positionen berechnet sind.

7. Verfahren (100) nach einem der Ansprüche 4 bis 6, wobei eine Aktion des Eingriffsplans dem Ermöglichen der Bewegung des Werkzeugs (15) in einem vorbestimmten Volumen im Verhältnis zur Anatomie von Interesse des Patienten (30) oder dem Ermöglichen der Bewegung des Werkzeugs (15) auf einer vorbestimmten Bewegungsbahn im Verhältnis zur Anatomie von Interesse des Patienten (30) oder dem Platzieren des Werkzeugs (15) in einer vorbestimmten Position im Verhältnis zur Anatomie von Interesse des Patienten (30) entspricht.

8. Verfahren (100) nach einem der Ansprüche 1 bis 7, wobei die optimale Position (73) je nach Einstellungen eines Bedieners auswählt wird, die in der Steuereinheit (16) gespeichert sind.

9. Verfahren (100) nach einem der Ansprüche 1 bis 8, wobei die optimale Position (73) von einem Algorithmus für maschinelles Lernen je nach Informationen ausgewählt wird, die aus einer Datenbank bereits vorgenommener medizinischer Eingriffe extrahiert werden.

10. Computerprogrammprodukt, **dadurch gekennzeichnet, dass** es einen Satz von Programmcode-Anweisungen aufweist, die bei Ausführung durch einen oder mehrere Prozessoren den oder die Prozessoren dafür konfigurieren, ein Verfahren (100) zur automatischen Positionierung eines medizinischen Roboters nach einem der Ansprüche 1 bis 9 durchzuführen.

11. Medizinischer Roboter (10), der einen motorisierten beweglichen Sockel (13), Sensoren (17) zur Positionsbestimmung im Raum, die fest mit dem beweglichen Sockel (13) verbunden sind, und eine Steuereinheit (16) aufweist, die einen Eingriffsplan speichert, der wenigstens eine Aktion aufweist, die an einer Anatomie von Interesse eines Patienten (30) durchzuführen ist, wobei die Steuereinheit (16) für Folgendes ausgebildet ist:
- Detektieren einer Position der Anatomie von Interesse des Patienten (30) im Verhältnis zum medizinischen Roboter (10) anhand von Informationen, die von den Sensoren (17) zur Positionsbestimmung im Raum stammen,
- Ermitteln, anhand der Position der Anatomie von Interesse des Patienten (30) und des Eingriffsplans, wenigstens zweier günstiger Positionen des beweglichen Sockels (13) des medizinischen Roboters (10), in denen der medizinische Roboter (10) imstande ist, die wenigstens eine Aktion des Eingriffsplans durchzuführen,
- Bewegen des beweglichen Sockels (13) des medizinischen Roboters (10) in eine aus den ermittelten günstigen Positionen ausgewählte optimale Position,
- Überprüfen eines Stabilitätskriteriums des beweglichen Sockels (13) des medizinischen Roboters (10) in der ausgewählten optimalen Position,
- wenn die Überprüfung positiv ist, Auslösen eines Festsetzens des beweglichen Sockels des medizinischen Roboters (10) in der optimalen Position,
- wenn die Überprüfung negativ ist, Bewegen des beweglichen Sockels (13) des medizinischen Roboters (10) in eine aus den günstigen Positionen ausgewählte andere Position,
**dadurch gekennzeichnet, dass** der medizinische Roboter (10) einziehbare Füße aufweist, die dazu bestimmt sind, abgesenkt zu werden, um den beweglichen Sockel (13) des medizinischen Roboters (10) festzusetzen, wobei jeder Fuß einen Sensor aufweist, mit dem ein Gewicht gemessen werden kann, das von dem Fuß getragen wird, und zum Überprüfen des Stabilitätskriteriums die Steuereinheit (16) dazu ausgebildet ist, für jeden Fuß die Messung des Gewichts, das von dem Fuß getragen wird, mit einer vorbestimmten Schwelle zu vergleichen.

12. Medizinischer Roboter (10) nach Anspruch 11, wobei die Steuereinheit (16) ebenfalls dazu ausgebildet ist, anhand von Informationen, die von den Sensoren (17) zur Positionsbestimmung im Raum stammen, eine Position des Tisches (40) eines Behandlungsraumes (50) zu detektieren, auf dem der Patient (30) gelagert ist, und die wenigstens eine günstige Position je nach der Position des Tisches (40) zu ermitteln.

13. Medizinischer Roboter (10) nach Anspruch 12, wobei die Steuereinheit (16) ebenfalls für Folgendes ausgebildet ist:
- Erzeugen einer Kartografie (70) des Behandlungsraumes (50), auf der eine Position (71) des medizinischen Roboters (10), die Position (72) des Tisches (40), die Position (75) der Anatomie von Interesse des Patienten (30) und die optimale Position (73) dargestellt sind,
- Bestimmen, anhand der Kartografie (70), eines Wegs (76) des medizinischen Roboters (10) zur Erreichung der optimalen Position (73).

14. Medizinischer Roboter (10) nach einem der Ansprüche 11 bis 13, wobei der medizinische Roboter (10) ferner Sensoren (20) zur Unterstützung bei der Detektion der Anatomie von Interesse des Patienten (30) aufweist, die mit Markern (19) zusammenwirken, die auf der Anatomie von Interesse positioniert sind, wobei die Sensoren (20) zur Unterstützung bei der Detektion der Anatomie von Interesse des Patienten (30) fest mit dem beweglichen Sockel (13) des medizinischen Roboters (10) verbunden sind, und wobei die Informationen, die von Sensoren stammen, die zur Ermittlung der günstigen Position(en) benutzt werden, ausschließlich von Sensoren bereitgestellt werden, die vom medizinischen Roboter getragen werden.

15. Medizinischer Roboter (10) nach einem der Ansprüche 11 bis 14, der einen Gelenkarm (14) und ein Werkzeug (15) aufweist, das an einem Ende des Arms (14) angebracht ist, wobei ein oder mehrere Gelenke des Arms (14) von der Steuereinheit (16) des medizinischen Roboters (10) gesteuert werden, um den Arm (14) entsprechend einer möglichen Konfiguration zu positionieren, wobei eine günstige Position einer Position des beweglichen Sockels (13) des medizinischen Roboters (10) entspricht, in der für jede Aktion des Eingriffsplans wenigstens eine mögliche Konfiguration des Gelenkarms (14) vorhanden ist, mit der die Aktion durchgeführt werden kann, und die Steuereinheit (16) ferner für Folgendes ausgebildet ist:
- Berechnen, für jede Aktion des Eingriffsplans und für jede ermittelte günstige Position, eines Erreichbarkeitsparameters, der für eine Anzahl von möglichen Konfigurationen des Gelenkarms (14) repräsentativ ist, um die Aktion durchzuführen, wenn der bewegliche Sockel (13) des medizinischen Roboters (10) in der günstigen Position positioniert ist,
- Auswählen der optimalen Position je nach den Erreichbarkeitsparametern der ermittelten günstigen Positionen.

## Claims

1. Method (100) for automatic positioning of a medical robot for carrying out a medical intervention on a patient (30), said medical robot (10) having a motorized mobile base (13) enabling movement of the medical robot (10), and a control unit (16) storing an intervention plan comprising at least one action to be performed on an anatomy of interest of said patient (30), said positioning method (100) comprising the following steps, executed autonomously by the medical robot (10):
- detection (101) of the position of the anatomy of interest of the patient (30) relative to a position of the medical robot (10),
- identification (102), on the basis of the position of the anatomy of interest of the patient (30) and of the intervention plan, of at least two different favourable positions of the mobile base (13) of the medical robot (10) at which the medical robot (10) is capable of performing said at least one action from the intervention plan,
- movement (106) of the mobile base (13) of the medical robot (10) to an optimum position selected from the identified favourable positions,
- verification of a stability criterion of the mobile base (13) of the medical robot (10) at the selected optimum position,
- if said verification is positive, immobilization of the mobile base (13) of the medical robot (10) at said optimum position,
- if said verification is negative, movement of the mobile base (13) of the medical robot (10) to another position selected from the favourable positions,
**characterized in that** the medical robot (10) has retractable feet intended to be lowered in order to immobilize the mobile base (13) of the medical robot (10), each foot having a sensor making it possible to measure a weight supported by said foot, and the verification of a stability criterion comprises, for each foot, comparing the measurement of the weight supported by said foot against a predetermined threshold.

2. Method (100) according to Claim 1, in which the patient (30) is placed on a table (40) of an operating room (50), the method (100) comprises detection of the position of the table (40), the identification (102) of the favourable positions of the mobile base (13) of the medical robot (10) is furthermore effected on the basis of the position of the table (40), and the detection of the position of the table (40) and the detection of the anatomy of interest of the patient (30) are effected on the basis of information from spatial location sensors (17) carried by the medical robot (10).

3. Method (100) according to Claim 2, in which the robot initially has no mapping of the operating room (50), and the method (100) further comprises the following steps executed autonomously by the medical robot (10):
- generation (104) of a mapping (70) of the operating room (50) on which are represented a position (71) of the medical robot (10), the position (72) of the table (40), the position (75) of the anatomy of interest of the patient (30), and the optimum position (73),
- determination (105), on the basis of said mapping (70), of a trajectory (76) of the medical robot (10) in order to reach said optimum position (73).

4. Method (100) according to one of Claims 1 to 3, in which:
- the medical robot (10) comprises an articulated arm (14) and a tool (15) mounted at one end of said arm (14), one or more articulations of said arm (14) being controlled by the control unit (16) of the medical robot (10) so as to position the arm (14) in accordance with one possible configuration,
- a favourable position corresponds to a position of the mobile base (13) of the medical robot (10) at which there exists, for each action from the intervention plan, at least one possible configuration of the articulated arm (14) enabling said action to be performed,
- the method (100) comprises a calculation (108), for each action from the intervention plan and for each identified favourable position, of an accessibility parameter as a function of a number of possible configurations of the articulated arm (14) for performing said action when the mobile base (13) of the medical robot (10) is positioned at said favourable position,
- the optimum position (73) is selected as a function of the accessibility parameters of the identified favourable positions.

5. Method (100) according to Claim 4, in which said accessibility parameter is furthermore calculated as a function of available clearance movements of the articulations of the arm (14) for each of said possible configurations of the arm (14).

6. Method (100) according to either of Claims 4 and 5, in which:
- the method (100) further comprises a calculation (109), for each identified favourable position, of a global level of accessibility of the intervention plan, said global level of accessibility being calculated as a function of the accessibility parameters of each action from the intervention plan when the mobile base (13) of the medical robot (10) is positioned at said favourable position,
- the optimum position (73) is selected as a function of the global levels of accessibility calculated for the identified favourable positions.

7. Method (100) according to one of Claims 4 to 6, in which an action from the intervention plan corresponds to enabling the movement of the tool (15) in a predetermined volume relative to said anatomy of interest of the patient (30), or enabling movement of the tool (15) with a predetermined trajectory relative to said anatomy of interest of the patient (30), or placing the tool (15) in a predetermined position relative to said anatomy of interest of the patient (30).

8. Method (100) according to one of Claims 1 to 7, in which the optimum position (73) is selected as a function of preferences of an operator that are stored in the control unit (16).

9. Method (100) according to one of Claims 1 to 8, in which the optimum position (73) is selected by an automatic learning algorithm as a function of information extracted from an existing medical intervention database.

10. Computer program product, **characterized in that** it comprises a set of program code instructions which, when executed by one or more processors, configure the processor or processors to execute a method (100) according to one of Claims 1 to 9 for automatic positioning of a medical robot.

11. Medical robot (10) comprising a motorized mobile base (13), spatial location sensors (17) fastened to the mobile base (13), and a control unit (16) storing an intervention plan comprising at least one action to be performed on an anatomy of interest of a patient (30), said control unit (16) being configured:
- to detect, on the basis of information from the spatial location sensors (17), a position of the anatomy of interest of the patient (30) relative to the medical robot (10),
- to identify, on the basis of the position of the anatomy of interest of the patient (30) and of the intervention plan, at least two favourable positions of the mobile base (13) of the medical robot (10) at which the medical robot (10) is capable of performing said at least one action from the intervention plan,
- to move the mobile base (13) of the medical robot (10) to an optimum position selected from the identified favourable positions,
- to verify a stability criterion of the mobile base (13) of the medical robot (10) at the selected optimum position,
- if said verification is positive, to trigger an immobilization of the mobile base of the medical robot (10) at said optimum position,
- if said verification is negative, to move the mobile base (13) of the medical robot (10) to another position selected from the favourable positions,
**characterized in that** the medical robot (10) has retractable feet intended to be lowered in order to immobilize the mobile base (13) of the medical robot (10), each foot having a sensor making it possible to measure a weight supported by said foot, and to verify the stability criterion, the control unit (16) is configured to compare, for each foot, the measurement of the weight supported by said foot against a predetermined threshold.

12. Medical robot (10) according to Claim 11, in which the control unit (16) is also configured to detect, on the basis of information from the spatial location sensors (17), a position of a table (40) of an operating room (50) on which the patient (30) is placed, and to identify said at least one favourable position as a function of the position of the table (40).

13. Medical robot (10) according to Claim 12, in which the control unit (16) is also configured:
- to generate a mapping (70) of the operating room (50) on which are represented a position (71) of the medical robot (10), the position (72) of the table (40), the position (75) of the anatomy of interest of the patient (30), and the optimum position (73),
- to determine, on the basis of said mapping (70), a trajectory (76) of the medical robot (10) in order to reach said optimum position (73) .

14. Medical robot (10) according to one of Claims 11 to 13, in which the medical robot (10) furthermore comprises sensors (20), for assisting the detection of the anatomy of interest of the patient (30), cooperating with markers (19) positioned on said anatomy of interest, the sensors (20) for assisting the detection of the anatomy of interest of the patient (30) being fastened to the mobile base (13) of the medical robot (10), and in which the information from sensors used to identify the favourable position or positions is furnished exclusively by sensors carried by the medical robot.

15. Medical robot (10) according to one of Claims 11 to 14, comprising an articulated arm (14) and a tool (15) mounted at one end of said arm (14), one or more articulations of said arm (14) being controlled by the control unit (16) of the medical robot (10) to position the arm (14) in accordance with one possible configuration, a favourable position corresponding to a position of the mobile base (13) of the medical robot (10) at which there exists, for each action from the intervention plan, at least one possible configuration of the articulated arm (14) enabling said action to be performed, and the control unit (16) is furthermore configured:
- to calculate, for each action from the intervention plan and for each identified favourable position, an accessibility parameter representing a number of possible configurations of the articulated arm (14) for performing said action when the mobile base (13) of the medical robot (10) is positioned at said favourable position,
- to select the optimum position as a function of the accessibility parameters of the identified favourable positions.
